# EUROPEAN PATENT APPLICATION

(11) **EP 4 112 125 A1**
(43) Date of publication of application: **04.01.2023**
(21) Application number: 22189792.9
(22) Date of filing: 12.03.2020
(51) Int. Cl.: A61P 35/00, A61K 39/395, C07K 16/28, A61K 39/00

(54) **METHODS AND COMPOSITIONS FOR TREATING CANCER**

(30) Priority: 12.03.2019 US 201962817231 P; 21.01.2020 US 202062963742 P
(62) Divisional of application: 20717476.4
(71) Applicant: President and Fellows of Harvard College, Cambridge, MA 02138 (US)
(72) Inventor: Gazzaniga, Francesca S., Jamaica Plain, 02130 (US); Park, Joon Seok, Brookline, 02445 (US); Sharpe, Arlene H., Brookline, 02445 (US); Kasper, Dennis, Brookline, 02445 (US)
(74) Representative: Hoefer & Partner Patentanwälte mbB

(57) **Abstract**

Provided herein are methods and compositions for treating cancer or a tumor in a subject by administering to the subject a first agent that disrupts the interaction between PD-L2/RGMb and a second agent that disrupts the interaction between PD-1/PD-L1. The subject may have dysbiosis and/or be nonresponsive to immune checkpoint therapy

## Description

### RELATED APPLICATIONS

This application claims priority to U.S. Provisional Application 62/817231, filed March 12, 2019, and U.S. Provisional Application 62/963742, filed January 21, 2020, each of which is incorporated herein by reference in its entirety.

### GOVERNMENT SUPPORT

This invention was made with government support under grant no. 5T32HD55148-10 awarded by the National Institutes of Health. The government has certain rights in the invention.

### BACKGROUND

Immune checkpoint blockade, or immunotherapy, is a novel therapeutic approach that reinvigorates tumor-specific T cells to efficiently kill cancer cells by blocking inhibitory pathways in T cells including CTLA-4 and PD-1. In recent years, antibodies against immune checkpoint molecules have attracted attention as new therapeutic agents for cancer. Immune checkpoint inhibitors promote the activation of T cells by inhibiting a molecule that suppresses the activation and function of T cells, and enhances the antitumor response of the T cells. In a treatment with an immune checkpoint inhibitor, cancer is eliminated by activating the immune state of the living body.

Despite the clinical success of immune checkpoint blockade-based drugs, a significant fraction of cancer patients do not respond to the therapy. Therefore, understanding the elements that regulate the efficacy of the checkpoint blockade is crucial to the development of more effective therapies.

### SUMMARY

A therapeutic method is described herein to convert non-responders to anti-PD-L1 therapy to responders by combination therapy with anti-PD-L1 and PD-L2 inhibitors. This disclosure also demonstrates a method to convert non-responders to anti-PD-1 therapy to responders by combination therapy with anti-PD-1 and PD-L2 inhibitors. This disclosure further demonstrates that the microbiome regulates PD-L2 expression, and shows the functional significance of PD-L2 in restraining anti-tumor immunity. This disclosure also demonstrates that disrupting the PD-L2/RGMb interaction enhances the efficacy of PD-1 or PD-L1 blockade.

Provided herein are methods and compositions for treating or preventing cancer in a subject. The methods described herein include administering to the subject a first agent that specifically binds to PD-L2 conjointly with a second agent that specifically binds to PD-1 or PD-L1. In some embodiments, the methods described herein comprise administering to the subject a first agent that specifically binds to RGMb conjointly with a second agent that specifically binds to PD-1 or PD-L1.

In some embodiments, the methods described herein comprise administering to the subject a first agent that disrupts RGMb, disrupts PD-L2, or disrupts the interaction between RGMb and PD-L2. In some embodiments, the first agent is administered conjointly with a second agent. The second agent may be an agent that disrupts PD-1, disrupts PD-L1, or disrupts the interaction between PD-1 and PD-L1. An agent may be an antibody or antigen binding fragment thereof, peptide, small molecule, or an inhibitory nucleic acid. For example, the first agent may be an antibody, and the antibody disrupts RGMb, disrupts PD-L2, or disrupts the interaction between RGMb and PD-L2.

In some embodiments, the methods include administering to the subject a first agent that disrupts the interaction between PD-L2/RGMb and a second agent that disrupts the interaction between PD-1/PD-L1. In additional embodiments, the methods include treating a patient that is dysbiotic with an agent that disrupts the interaction between PD-L2/RGMb. In further embodiments, the methods include treating a patient that is a non-responder to PD-1/PD-L1 blockade therapy with an agent that disrupts the interaction between PD-L2/RGMb.

The first agent and second agent may be any agent described herein (e.g., an antibody). The first agent may be an antibody that disrupts RGMb, an antibody that blocks PD-L2, or an antibody that disrupts the interaction between the RGMb and PD-L2. The antibody may be a monoclonal antibody, a humanized antibody, and/or a bispecific antibody.

As used herein, an agent that "disrupts" a molecule includes any agent that lowers levels of the molecule, depletes cells that express the molecule, or blocks or lowers the molecule's activity. Disrupting a molecule also includes disrupting the interaction between the molecule and its ligands. Disrupting the interaction between two molecules (e.g., between PD-L2/RGMb or PD-1/PD-L1) includes contacting the target molecule(s) or cells expressing the target molecule(s) with an agent that modulates, alters, or blocks the interaction between the two molecules. Disrupting the interaction between two molecules includes contacting the cells expressing one of the molecules with an agent that depletes the cells expressing the molecule. For example, disrupting the interaction between two molecules with an antibody includes contacting target cells expressing at least one of the molecules with a blocking antibody, a neutralizing antibody, or a depleting antibody. Disrupting the interaction between RGMb and PD-L2 includes contacting target cells with an agent that blocks the interaction or an agent that depletes cells expressing RGMb.

The second agent may be an antibody, such as an antibody that blocks PD-1, an antibody that blocks PD-L1, an antibody that disrupts the interaction between PD-1 and its ligands or an antibody that disrupts the interaction between PD-L1 and its ligands. The antibody may be a monoclonal antibody, a humanized antibody, and/or a bispecific antibody.

The antibody that blocks PD-1 may be selected from cemiplimab (REGN2810), nivolumab (BMS-936558, MDX-1106, ONO-4538), pembrolizumab (MK-3475, SCH 900475), SHR1210, sintilimab (IBI308), spartalizumab (PDR001), tislelizumab (BGB-A317), pidilizumab, BCD-100, toripalimab (JS001), PF-06801591, AB122, AK105, AMG 404, BCD-100, BI 754091, F520, HLX10, HX008, JTX-4014, LZM009, MEDI0680, MGA012, Sym021, TSR-042, PSB205, MGD019, MGD013, AK104, XmAb20717, RO7121661, and CX-188.

The antibody that blocks PD-L1 may be selected from atezolizumab (MPDL3280A, RG7446, RO5541267), durvalumab (MEDI4736, MEDI-4736), avelumab (MSB0010718C), FS118, BCD-135, BGB-A333, CBT-502, CK-301, CS1001, FAZ053, HLX20, KN035, MDX-1105, MSB2311, SHR-1316, TG-1501, ZKAB001, INBRX-105, MCLA-145, KN046, M7824, and LY3415244.

The first and/or second agent may be administered systemically, orally, parenterally, or intravenously.

In some embodiments, the subject is nonresponsive to immune checkpoint inhibitor therapy. In other embodiments, the subject is nonresponsive to anti-PD-1 therapy or anti-PD-L1 therapy. In some embodiments, at least one antibiotic (e.g., at least two, at least three, at least four, at least five, at least six, at least seven, at least eight, at least nine or at least ten antibiotics) was administered to the subject within a period of time prior to administration of the first agent. The at least one antibiotic may be Vancomycin, Neomycin, Metronidazole and/or Ampicillin. The period of time may be one day, one week, two weeks, three weeks, one month, two months, three months, four months, five months, six months, nine months, one year, two years, three years, four years or five years.

In some embodiments, the subject has dysbiosis. Dysbiosis refers to any altered state of microbiota of the gastrointestinal (GI) tract. As used herein, "dysbiosis" includes patients that have been treated with antibiotics, received chemotherapy, or have or have had conditions known to alter the microbiome such as: intestinal infections, ulcerative colitis, Crohn's disease, irritable bowel syndrome, colon cancer, dramatic changes or significant changes in diet (for example extended hospital stays). In a normal distribution of bacterial phlya in the gut, Bacteroidetes and Firmicutes are dominant. In some embodiments, a patient has dysbiosis if the gastrointestinal microbiota of the subject is comprised dominantly of E. coli or bacteria from other phyla instead of being dominantly comprised of Bacteroidetes and/or Firmicutes bacteria. Additionally, patients have dysbiosis if they have had their microbiome sequenced at different time points and the microbiome makeup has changed. For example, the gastrointestinal microbiota of the subject may comprise at least 50%, at least 60%, at least 70%, at least 80%, or at least 90% E. coli or other bacteria from other phyla that is not Bacteroidetes and/or Firmicutes. The gastrointestinal microbiota of the subject may have an imbalance of the normal distribution of bacterial phyla in the gut. Dysbiosis includes any microbiota profile typical of a patient with cancer, and/or a microbiota profile not typically seen in patients without cancer. For example, imbalance in the GI tract of a subject with dysbiosis includes a higher level of bacteria that are not Bacteroidetes and Firmicutes in the GI tract of the subject when compared to the level of other phyla that is not Bacteroidetes and Firmicutes in the GI tract of a subject without cancer, or when compared to the average or median level of other phyla that is not Bacteroidetes and Firmicutes in the GI tract of a population of subjects without cancer. Dysbiosis may also refer to a lack of microbiota diversity in the GI tract. Dysbiosis includes the altered GI microbiota typically found in an individual after antibiotic administration. Dysbiotic patients may be identified, for example, by the symptoms of dysbiosis, such as diarrhea, constipation, abdominal cramping, loose stool, and/or abnormal amounts of gas or bloating. Some patients may be assumed to be dysbiotic because of previous administration of cancer or antibiotic treatments known to cause dysbiosis.

It should be appreciated that conjoint therapies disclosed herein would be effective in treating a variety of cancers and tumors. For example, the cancer may be lung cancer, a breast cancer, a colon cancer, a cervical cancer, a pancreatic cancer, a renal cancer, a stomach cancer, a GI cancer, a liver cancer, a bone cancer, a hematological cancer, a neural tissue cancer, a melanoma, a thyroid cancer, a ovarian cancer, a testicular cancer, a prostate cancer, a cervical cancer, a vaginal cancer, or a bladder cancer.

The cancer may comprise a tumor, and the tumor may be an adenocarcinoma, an adrenal tumor, an anal tumor, a bile duct tumor, a bladder tumor, a bone tumor, a brain/CNS tumor, a breast tumor, a cervical tumor, a colorectal tumor, an endometrial tumor, an esophageal tumor, an Ewing tumor, an eye tumor, a gallbladder tumor, a gastrointestinal, a kidney tumor, a laryngeal or hypopharyngeal tumor, a liver tumor, a lung tumor, a mesothelioma tumor, a multiple myeloma tumor, a muscle tumor, a nasopharyngeal tumor, a neuroblastoma, an oral tumor, an osteosarcoma, an ovarian tumor, a pancreatic tumor, a penile tumor, a pituitary tumor, a primary tumor, a prostate tumor, a retinoblastoma, a Rhabdomyosarcoma, a salivary gland tumor, a soft tissue sarcoma, a melanoma, a metastatic tumor, a basal cell carcinoma, a Merkel cell tumor, a testicular tumor, a thymus tumor, a thyroid tumor, a uterine tumor, a vaginal tumor, a vulvar tumor, or a Wilms tumor.

In some embodiments, an additional agent is administered, such as a chemotherapeutic agent or an additional immune checkpoint inhibitor. The additional immune checkpoint inhibitor may comprise an antibody or agent specific for an immune checkpoint protein selected from CTLA-4, VISTA, B7-H2, B7-H3, B7-H4, B7-H6, ICOS, HVEM, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, HHLA2, butyrophilins, and A2aR.

### BRIEF DESCRIPTION OF THE DRAWINGS

This patent or application file contains at least one drawing executed in color. Copies of this patent or patent application publication with color drawings will be provided by the Office upon request and payment of necessary fee.
**Figure 1** shows that mice treated with broad spectrum antibiotics (VNMA) for 17 days have an altered microbiota, referred to as dysbiosis. This microbiota is made up dominantly a Proteobacteria, E. coli. Mice treated with VNMA, but then given an oral dose of a healthy human microbiota (Hmb) have a much more diverse microbiota. Each color represents a different species defined by 16S sequencing. Yellow represents E. coli.
**Figure 2** shows that mice treated with VNMA do not respond to anti-PD-Ll therapy, whereas mice given an oral dose of Hmb are able to clear tumors with anti-PD-Ll therapy indicating that dysbiosis caused by VNMA treatment blocks the anti-tumor effects of ant-PD-L1 therapy.
**Figure 3** consists of two parts, A-B, and shows that 10 days (A) and 13 days (B) after tumor implantation, Hmb mice express significantly lower levels of anti-PD-L2 on macrophages and dendritic cells compared to VNMA mice in the tumor draining lymph nodes.
**Figure 4** shows that VNMA-treated mice do not respond to anti-PD-Ll or anti-PD-L2 alone. However, when given in combination, anti-PD-Ll and anti-PD-L2 therapy reverse the effects of dysbiosis and promote an anti-tumor response.

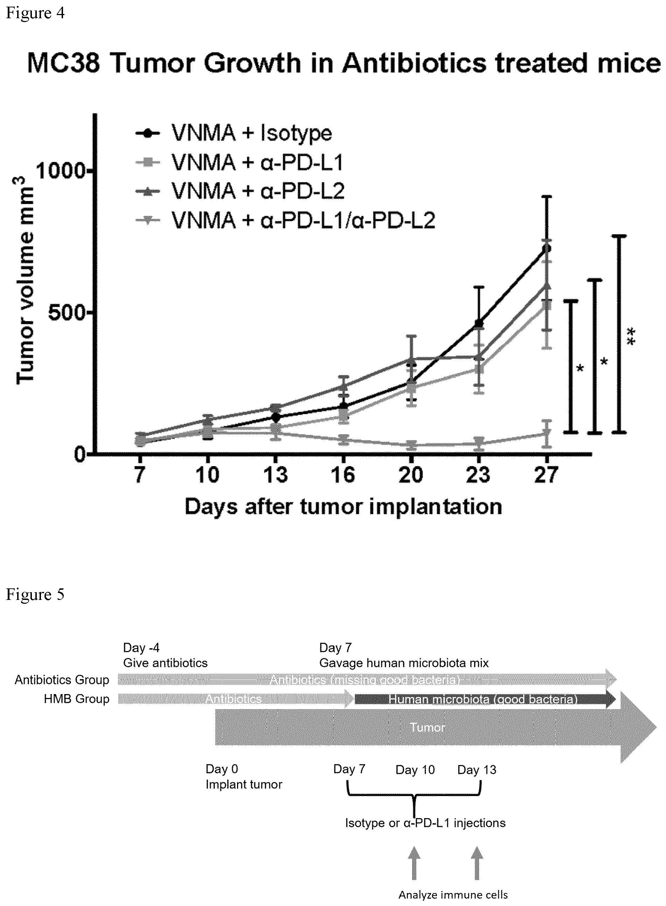

**Figure 5** shows the process for identifying the mechanisms by which good bacteria promote anti-tumor immunity. Mice are given antibiotics (0.5 mg/ml Vancomycin, 1 mg/ml Neomycin, 1mg/ml metronidazole, 1mg/ml Ampicillin) in drinking water 4 days before tumor implantation. On day zero, 2.5 × 10⁵ MC38 tumor cells are implanted subcutaneously in the abdomen of 6 week old female mice. On days 7, 10, 13, 16 mice are treated with 100 µg of isotype control or anti-PD-Ll by intraperitoneal injection. On day 7, half of the mice are orally gavaged with a slurry of Hmb feces and antibiotics are removed from the drinking water; the other half of the mice do not receive Hmb feces and continue with antibiotics in the drinking water for the remainder of the experiment. Tumors are measured on days 7, 10, 13, 16, 20, 23.
**Figure 6** shows that combined PD-L1 and PD-L2 blockade synergistically promotes anti-tumor response in germ-free (GF) mice.
**Figure 7** shows that blocking PD-L2 mediated signaling pathways enhances αPD-1 induced anti-tumor responses in germ-free mice.
**Figure 8** shows that αPD-1 and αRGMb synergistically promotes anti-tumor response to immunotherapy in germ-free mice.
**Figure 9** shows that αPD-L1 and αRGMb synergistically promotes anti-tumor response to immunotherapy in germ-free mice.

### DETAILED DESCRIPTION

Provided herein are methods and compositions for treating or preventing cancer in a subject. The methods described herein include administering to the subject a first agent that specifically binds to PD-L2 conjointly with a second agent that specifically binds to PD-1 or PD-L1. In some embodiments, the methods described herein comprise administering to the subject a first agent that specifically binds to RGMb conjointly with a second agent that specifically binds to PD-1 or PD-L1. In some embodiments, the methods described herein comprise administering to the subject a first agent that disrupts RGMb, disrupts PD-L2, or disrupts the interaction between RGMb and PD-L2.

The subject may have dysbiosis. In some embodiments, the subject is nonresponsive to anti-PD-1 or anti-PD-L1 therapy. In some embodiments, the subject has dysbiosis and is nonresponsive to anti-PD-1 or anti-PD-Ll therapy.

As used herein, "anti-PD-1 therapy" or "anti-PD-Ll therapy" includes cancer treatment regimes that lack administration of agents that disrupt the interaction between RGMb and PD-L2 or specifically bind and block RGMb or PD-L2. Patients undergoing anti-PD-1 therapy or anti-PD-Ll therapy may, for example, be undergoing other cancer treatments that do not include agents that disrupt the interaction between RGMb and PD-L2 or specifically bind and block RGMb or PD-L2.

As used herein, the term "nonresponsive" or "non-responders" includes patients that are refractory or resistant to previous treatments (e.g., refractory or resistant to PD-1 and/or PD-L1 immune checkpoint blockade). Nonresponsive patients include any patient who was subjected to PD-1 or PD-L1 blockade therapy and did not exhibit parameters of cancer regression or slowing of tumor progression. Nonresponsive patients include any patient that has been deemed clinically nonresponsive to PD-1 or PD-L1 blockade, or otherwise exhibited a poor clinical response to PD-1 or PD-L1 blockade. In some embodiments, a nonresponsive patient includes patients that initially respond to PD-1 or PD-L1 blockade therapy but develop resistance to such therapies. Also provided herein are methods of preventing or treating anti-PD-1/anti-PD-L1 resistance in a subject by administering to the subject an agent disrupts the interaction between RGMb and PD-L2 or specifically binds and blocks RGMb or PD-L2.

In one aspect, the methods provided herein include methods of treating or preventing cancer in a subject by administering to the subject a first agent that disrupts the interaction between RGMb and PD-L2 or specifically bind and block RGMb or PD-L2 and a second agent that specifically binds to PD-1 or PD-L1.

The invention described herein is based, in part, on the discovery that dysbiotic patients exhibit high expression levels of anti-PD-L2, and that PD-L2 blockade can overcome resistance to anti-PD-1 or anti-PD-Ll therapy. The invention described herein is further based, in part, on the discovery that PD-L2 binds to RGMb. Provided herein are methods of treating or preventing cancer in a subject by administering to the subject a first agent that specifically binds to PD-L2 or RGMb such that the interaction between PD-L2 and RGMb is disrupted conjointly with a second agent that specifically binds to PD-1 or PD-L1 such that the interaction between PD-1 and PD-L1 is disrupted. In other aspects, the methods provided herein include methods of treating or preventing cancer in a subject by administering to the subject a first agent that disrupts the interaction between RGMb and PD-L2 conjointly with a second agent that specifically binds to PD-1 or PD-L1 such that the interaction between PD-1 and PD-L1 is disrupted. The agent may be an antibody, such as a monoclonal or humanized antibody.

### Definitions

For convenience, certain terms employed in the specification, examples, and appended claims are collected here.

As used herein, the term *"administering"* means providing a pharmaceutical agent or composition to a subject, and includes, but is not limited to, administering by a medical professional and self-administering.

The term "*agent*" is used herein to denote a chemical compound, a small molecule, a mixture of chemical compounds and/or a biological macromolecule (such as an antibody, nucleic acid, a protein, or a peptide). Agents may be identified as having a particular activity by screening assays described herein below. The activity of such agents may render them suitable as a "therapeutic agent" which is a biologically, physiologically, or pharmacologically active substance (or substances) that acts locally or systemically in a subj ect.

The term *"amino acid*" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing.

As used herein, the term *"antibody"* may refer to both an intact antibody and an antigen-binding fragment thereof. Intact antibodies are glycoproteins that include at least two heavy (H) chains and two light (L) chains inter-connected by disulfide bonds. Each heavy chain includes a heavy chain variable region (abbreviated herein as V_{H}) and a heavy chain constant region. Each light chain includes a light chain variable region (abbreviated herein as V_{L}) and a light chain constant region. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. The term "antibody" includes, for example, monoclonal antibodies, polyclonal antibodies, chimeric antibodies, humanized antibodies, human antibodies, multi-specific antibodies (e.g., bispecific antibodies), single-chain antibodies and antigen-binding antibody fragments. An *"isolated antibody,"* as used herein, refers to an antibody which is substantially free of other antibodies having different antigenic specificities. An isolated antibody may, however, have some cross-reactivity to other, related antigens.

The terms *"antigen-binding fragment*" and *"antigen-binding portion"* of an antibody, as used herein, refers to one or more fragments of an antibody that retain the ability to bind to an antigen. Examples of binding fragments encompassed within the term "antigen-binding fragment" of an antibody include Fab, Fab', F(ab')₂, Fv, scFv, disulfide linked Fv, Fd, diabodies, single-chain antibodies, NANOBODIES^{®}, isolated CDRH3, and other antibody fragments that retain at least a portion of the variable region of an intact antibody. These antibody fragments can be obtained using conventional recombinant and/or enzymatic techniques and can be screened for antigen binding in the same manner as intact antibodies.

The terms *"CDR",* and its plural *"CDRs",* refer to a complementarity determining region (CDR) of an antibody or antibody fragment, which determine the binding character of an antibody or antibody fragment. In most instances, three CDRs are present in a light chain variable region (CDRL1, CDRL2 and CDRL3) and three CDRs are present in a heavy chain variable region (CDRH1, CDRH2 and CDRH3). CDRs contribute to the functional activity of an antibody molecule and are separated by amino acid sequences that comprise scaffolding or framework regions. Among the various CDRs, the CDR3 sequences, and particularly CDRH3, are the most diverse and therefore have the strongest contribution to antibody specificity. There are at least two techniques for determining CDRs: (1) an approach based on cross-species sequence variability (*i.e.,* Kabat et al., Sequences of Proteins of Immunological Interest (National Institute of Health, Bethesda, Md. (1987), incorporated by reference in its entirety); and (2) an approach based on crystallographic studies of antigen-antibody complexes (Chothia et al., Nature, 342:877 (1989), incorporated by reference in its entirety).

As used herein, the term *"humanized antibody"* refers to an antibody that has at least one CDR derived from a mammal other than a human, and a FR region and the constant region of a human antibody. A humanized antibody is useful as an effective component in a therapeutic agent since antigenicity of the humanized antibody in human body is lowered.

As used herein, the term *"monoclonal antibody"* refers to an antibody obtained from a population of substantially homogeneous antibodies that specifically bind to the same epitope, *i.e.,* the individual antibodies comprising the population are identical except for possible naturally occurring mutations that may be present in minor amounts. The modifier "monoclonal" indicates the character of the antibody as being obtained from a substantially homogeneous population of antibodies, and is not to be construed as requiring production of the antibody by any particular method.

The terms *"polynucleotide",* and *"nucleic acid*" are used interchangeably. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component. The term "recombinant" polynucleotide means a polynucleotide of genomic, cDNA, semisynthetic, or synthetic origin which either does not occur in nature or is linked to another polynucleotide in a non-natural arrangement.

The phrase *"pharmaceutically-acceptable carrier"* as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body.

As used herein, a therapeutic that *"prevents"* a disorder or condition refers to a compound that, when administered to a statistical sample prior to the onset of the disorder or condition, reduces the occurrence of the disorder or condition in the treated sample relative to an untreated control sample, or delays the onset or reduces the severity of one or more symptoms of the disorder or condition relative to the untreated control sample.

As used herein, *"specific binding*" refers to the ability of an antibody to bind to a predetermined antigen or the ability of a polypeptide to bind to its predetermined binding partner. Typically, an antibody or polypeptide specifically binds to its predetermined antigen or binding partner with an affinity corresponding to a K_{D} of about 10⁻⁷ M or less, and binds to the predetermined antigen/binding partner with an affinity (as expressed by K_{D}) that is at least 10 fold less, at least 100 fold less or at least 1000 fold less than its affinity for binding to a non-specific and unrelated antigen/binding partner (e.g., BSA, casein).

The term *"small molecule"* is a term of the art and includes molecules that are less than about 1000 molecular weight or less than about 500 molecular weight. In one embodiment, small molecules do not exclusively comprise peptide bonds. In another embodiment, small molecules are not oligomeric. Exemplary small molecule compounds which can be screened for activity include, but are not limited to, peptides, peptidomimetics, nucleic acids, carbohydrates, small organic molecules *(e.g.,* polyketides) (Cane et al. (1998) Science 282:63), and natural product extract libraries. In another embodiment, the compounds are small, organic non-peptidic compounds. In a further embodiment, a small molecule is not biosynthetic.

As used herein, the term "*subject*" means a human or non-human animal selected for treatment or therapy.

The *"tumor microenvironment*" is an art-recognized term and refers to the cellular environment in which the tumor exists, and includes, for example, interstitial fluids surrounding the tumor, surrounding blood vessels, immune cells, other cells, fibroblasts, signaling molecules, and the extracellular matrix.

The phrases *"therapeutically-effective amount"* and *"effective amount*" as used herein means the amount of an agent which is effective for producing the desired therapeutic effect in at least a sub-population of cells in a subject at a reasonable benefit/risk ratio applicable to any medical treatment.

"*Treating*" a disease in a subject or "*treating*" a subject having a disease refers to subjecting the subject to a pharmaceutical treatment, e.g., the administration of a drug, such that at least one symptom of the disease is decreased or prevented from worsening.

### Therapeutic Methods

Provided herein are methods of treating or preventing cancer in a subject, comprising administering to the subject a first agent that disrupts the PD-L2/RGMb interaction and a second agent that disrupts the PD-1/PD-L1 interaction. In one aspect, the methods provided herein include methods of treating or preventing cancer in a subject, comprising administering to the subject a first agent that specifically binds to PD-L2 conjointly with a second agent that specifically binds to PD-1 or PD-L1. In other aspects, the methods provided herein include methods of treating or preventing cancer in a subject by administering to the subject a first agent that specifically binds to RGMb conjointly with a second agent that specifically binds to PD-1 or PD-L1.

In some embodiments, the subject is nonresponsive to immune checkpoint inhibitor therapy (e.g., anti-PD-1 and/or anti-PD-Ll therapy).

In some embodiments, the subject has received at least one antibiotic administered within a period of time prior to administration of the first agent. The at least one antibiotic may be any antibiotic that causes dysbiosis in the gastrointestinal tract when administered (e.g., self-administered or administered by another person) to a patient. The antibiotic may be any antibiotic that causes dysbiosis by disrupting the balance of normal microbiota in the gastrointestinal tract. The antibiotic may be, but is not limited to, Vancomycin, Neomycin, Metronidazole, or Ampicillin. The period of antibiotic administration may have occurred 1 day, 1 week, three weeks, one month, three months, six months, nine months, one year, two years, three years, four years, or five years ago. Dysbiosis, as used herein, is not limited solely to patients who have received antibiotic treatment. Dysbiotic patients may also be identified, for example, by the symptoms of dysbiosis, such as diarrhea, constipation, abdominal cramping, loose stool, and/or abnormal amounts of gas or bloating. Some patients may be assumed to be dysbiotic because of previous administration of cancer treatments known to cause dysbiosis.

The agents (e.g., the first and/or second agents, or any additional agent) and/or compositions described herein may be administered systemically, intravenously, intramuscularly, orally, or locally (e.g., delivered locally to a tumor). The compositions and/or agents disclosed herein may be delivered by any suitable route of administration, including orally, nasally, as by, for example, a spray, rectally, intravaginal, parenterally, intracisternally and topically, as by powders, ointments or drops, including buccally and sublingually. In certain embodiments, the agents and/or compositions are delivered generally (e.g., via oral or parenteral administration). In certain other embodiments, the compositions and/or agents are delivered locally through injection. The therapeutics described herein may be administered through conjunctive therapy. Conjunctive therapy includes sequential, simultaneous and separate, and/or co-administration of the compositions and/or agents in such a way that the therapeutic effects of the first agent administered have not entirely disappeared when the subsequent agent is administered. In certain embodiments, the additional agent may be co-formulated with the first and/or second agent or be formulated in a separate pharmaceutical composition. In some embodiments, the compositions and additional agents are administered at the same time or at different times (e.g., the compositions and additional agents are administered sequentially).

Typical subjects for treatment include persons afflicted with or suspected of having or being pre-disposed to a disease disclosed herein, or persons susceptible to, suffering from or that have suffered a disease disclosed herein. A subject may or may not have a genetic predisposition for a disease disclosed herein. As used herein, the phrase "conjoint administration" refers to any form of administration of two or more different agents (e.g., two different antibodies) such that the second agent is administered while the previously administered agent is still effective in the body. For example, the compositions disclosed herein can be administered either in the same formulation or in a separate formulation, either concomitantly or sequentially.

The pharmaceutical compositions disclosed herein may be delivered by any suitable route of administration, including orally, locally, and parenterally. In certain embodiments the pharmaceutical compositions are delivered generally (e.g., via oral or parenteral administration).

In certain aspects, agents and/or compositions disclosed herein may be administered at a dose sufficient to achieve the desired result.

In certain embodiments, the method may comprise administering about 1 (µg to about 1 gram of agent or composition to the subject, such as about 1 µg to about 1 mg, about 2 µg to about 2 mg, about 3 µg to about 3 mg, about 4 µg to about 4 mg, about 100 µg to about 2 mg, about 200 µg to about 2 mg, about 300 µg to about 3 mg, about 400 µg to about 4 mg, about 250 µg to about 1 mg, or about 250 µg to about 750 µg of the agent or composition. In some embodiments, the method may comprise administering about 25 µg, about 50 µg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 600 µg/kg, about 650 µg/kg, about 700 µg/kg, about 750 µg/kg, about 800 µg/kg, about 850 µg/kg, about 900 µg/kg, about 950 µg/kg, about 1000 µg/kg, about 1200 µg/kg, about 1250 µg/kg, about 1300 µg/kg, about 1333 µg/kg, about 1350 µg/kg, about 1400 µg/kg, about 1500 µg/kg, about 1600 µg/kg, about 1750 µg/kg, about 1800 µg/kg, about 2000 µg/kg, about 2200 µg/kg, about 2250 µg/kg, about 2300 µg/kg, about 2333 µg/kg, about 2350 µg/kg, about 2400 µg/kg, about 2500 µg/kg, about 2667 µg/kg, about 2750 µg/kg, about 2800 µg/kg, about 3 mg/kg, about 3.5 mg/kg, about 3.5 mg/kg, about 4 mg/kg, about 4.5 mg/kg, about 5 mg/kg, about 6 mg/kg, about 7 mg/kg, about 8 mg/kg, about 9 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, or about 100 mg/kg. In some embodiments, the method may comprise administering about 1 mg/kg to about 10 mg/kg, about 10 mg/kg to about 20 mg/kg, about 20 mg/kg to about 50 mg/kg, about 50 mg/kg to about 100 mg/kg of the agent or composition. The dose may be titrated up or down following initial administration to any effective dose.

Immune checkpoint inhibitor dosing may follow any dosing regime or schedule known in the art. For example, dosing can be determined by cancer type or cancer disease stage, as well as the characteristics of the afflicted patient, such as weight, sex, ethnicity, and/or sensitivity to medication. Exemplary dosing regimes and schedules can be found at https://packageinserts.bms.com/pi/pi_opdivo.pdf; https://www.merck.com/product/usa/pi_circulars/k/keytruda/keytruda_pi.pdf; https://www.accessdata.fda.gov/drugsatfda_docs/label/2018/761069s002lbl.pdf; or https://www.accessdata.fda.gov/drugsatfda_docs/label/2017/761049s000lbl.pdf.

In some embodiments, administering an agent (e.g., a first and/or second agent) or composition to the subject comprises administering a bolus of the composition. The method may comprise administering the composition to the subject at least once per month, twice per month, three times per month. In certain embodiments, the method may comprise administering the composition at least once per week, at least once every two weeks, or once every three weeks. In some embodiments, the method may comprise administering the composition to the subject 1, 2, 3, 4, 5, 6, or 7 times per week.

### Antibody Agents

In certain embodiments, the methods and compositions provided herein relate to antibodies and antigen binding fragments thereof that bind specifically to PD-L2 (e.g., a first agent), RGMb (e.g., a first agent), PD-1 (e.g., a second agent) or PD-L1 (e.g., a second agent). In some embodiments, the antibodies disrupt the molecules disclosed herein. Such antibodies can be polyclonal or monoclonal and can be, for example, murine, chimeric, humanized or fully human. The antibody may be bispecific (e.g., bispecific for PD-L2 and RGMb). Exemplary PD-L2 antibodies can be found in patents and published applications such as: US Patent No. 9,845,356, US Patent No. 10,370,448, US Patent Publication 2018/0002422, WO Pat. Publication WO2002000730, and US Patent Publication 2018/0258171, hereby incorporated by reference in their entireties. Exemplary PD-L1 or PD-1 antibodies include, without limitation, cemiplimab (REGN2810), nivolumab (BMS-936558, MDX-1106, ONO-4538), pembrolizumab (MK-3475, SCH 900475), SHR1210, sintilimab (IBI308), spartalizumab (PDR001), tislelizumab (BGB-A317), pidilizumab, BCD-100, toripalimab (JS001), PF-06801591, AB122, AK105, AMG 404, BCD-100, BI 754091, F520, HLX10, HX008, JTX-4014, LZM009, MEDI0680, MGA012, Sym021, TSR-042, PSB205, MGD019, MGD013, AK104, XmAb20717, RO7121661, CX-188, atezolizumab (MPDL3280A, RG7446, RO5541267), durvalumab (MEDI4736, MEDI-4736), avelumab (MSB0010718C), FS118, BCD-135, BGB-A333, CBT-502, CK-301, CS1001, FAZ053, HLX20, KN035, MDX-1105, MSB2311, SHR-1316, TG-1501, ZKAB001, INBRX-105, MCLA-145, KN046, M7824, and LY3415244.

Polyclonal antibodies can be prepared by immunizing a suitable subject (e.g., a mouse) with a polypeptide antigen (e.g., a polypeptide having a sequence of PD-L2, RGMb, or a fragment thereof). The polypeptide antibody titer in the immunized subject can be monitored over time by standard techniques, such as with an enzyme linked immunosorbent assay (ELISA) using immobilized polypeptide. If desired, the antibody directed against the antigen can be isolated from the mammal (e.g., from the blood) and further purified by well-known techniques, such as protein A chromatography to obtain the IgG fraction.

At an appropriate time after immunization, e.g., when the antibody titers are highest, antibody-producing cells can be obtained from the subject and used to prepare monoclonal antibodies using standard techniques, such as the hybridoma technique originally described by Kohler and Milstein (1975) Nature 256:495-497) (see also Brown et al. (1981) J. Immunol. 127:539-46; Brown et al. (1980) J. Biol. Chem. 255:4980-83; Yeh et al. (1976) Proc. Natl. Acad. Sci. 76:2927-31; and Yeh et al. (1982) Int. J. Cancer 29:269-75), the more recent human B cell hybridoma technique (Kozbor et al. (1983) Immunol. Today 4:72), the EBV-hybridoma technique (Cole et al. (1985) Monoclonal Antibodies and Cancer Therapy, Alan R. Liss, Inc., pp. 77-96) or trioma techniques. The technology for producing monoclonal antibody hybridomas is well known (see generally Kenneth, R. H. in Monoclonal Antibodies: A New Dimension In Biological Analyses, Plenum Publishing Corp., New York, New York (1980); Lerner, E. A. (1981) Yale J. Biol. Med. 54:387-402; Gefter, M. L. et al. (1977) Somatic Cell Genet. 3:231-36). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds to the polypeptide antigen, preferably specifically.

As an alternative to preparing monoclonal antibody-secreting hybridomas, a monoclonal specific for a receptor or ligand provided herein can be identified and isolated by screening a recombinant combinatorial immunoglobulin library (e.g., an antibody phage display library or an antibody yeast display library) with the appropriate polypeptide to thereby isolate immunoglobulin library members that bind the polypeptide.

Additionally, recombinant antibodies specific for a receptor or ligand provided herein, such as chimeric or humanized monoclonal antibodies, can be made using standard recombinant DNA techniques. Such chimeric and humanized monoclonal antibodies can be produced by recombinant DNA techniques known in the art, for example using methods described in US Pat No. 4,816,567; US Pat. No. 5,565,332; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. 84:214-218; Nishimura et al. (1987) Cancer Res. 47:999-1005; Wood et al. (1985) Nature 314:446-449; and Shaw et al. (1988) J. Natl. Cancer Inst. 80:1553-1559); Morrison, S. L. (1985) Science 229:1202-1207; Oi et al. (1986) Biotechniques 4:214; Winter U.S. Patent 5,225,539; Jones et al. (1986) Nature 321:552-525; Verhoeyan et al. (1988) Science 239:1534; and Beidler et al. (1988) J Immunol. 141:4053-4060.

Human monoclonal antibodies specific for a receptor or ligand provided herein can be generated using transgenic or transchromosomal mice carrying parts of the human immune system rather than the mouse system. For example, "HuMAb mice" which contain a human immunoglobulin gene miniloci that encodes unrearranged human heavy (µ and γ) and κ light chain immunoglobulin sequences, together with targeted mutations that inactivate the endogenous µ and κ chain loci (Lonberg, N. et al. (1994) Nature 368(6474): 856 859). Accordingly, the mice exhibit reduced expression of mouse IgM or κ, and in response to immunization, the introduced human heavy and light chain transgenes undergo class switching and somatic mutation to generate high affinity human IgGκ monoclonal antibodies (Lonberg, N. *et al.* (1994), supra; reviewed in Lonberg, N. (1994) Handbook of Experimental Pharmacology 113:49 101; Lonberg, N. and Huszar, D. (1995) Intern. Rev. Immunol. Vol. 13: 65 93, and Harding, F. and Lonberg, N. (1995) Ann. N. Y Acad. Sci 764:536 546). The preparation of HuMAb mice is described in Taylor, L. et al. (1992) Nucleic Acids Research 20:6287 6295; Chen, J. et al. (1993) International Immunology 5: 647 656; Tuaillon et al. (1993) Proc. Natl. Acad. Sci USA 90:3720 3724; Choi et al. (1993) Nature Genetics 4:117 123; Chen, J. et al. (1993) EMBO J. 12: 821 830; Tuaillon et al. (1994) J. Immunol. 152:2912 2920; Lonberg et al., (1994) Nature 368(6474): 856 859; Lonberg, N. (1994) Handbook of Experimental Pharmacology 113:49 101; Taylor, L. et al. (1994) International Immunology 6: 579 591; Lonberg, N. and Huszar, D. (1995) Intern. Rev. Immunol. Vol. 13: 65 93; Harding, F. and Lonberg, N. (1995) Ann. N.Y. Acad. Sci 764:536 546; Fishwild, D. et al. (1996) Nature Biotechnology 14: 845 851. See further, U.S. Pat. Nos. 5,545,806; 5,569,825; 5,625,126; 5,633,425; 5,789,650; 5,877,397; 5,661,016; 5,814,318; 5,874,299; 5,770,429; and 5,545,807.

### Proteins

In certain embodiments, the compositions and methods provided herein relate to polypeptides that specifically bind to PD-L2, RGMb, PD-1, or PD-L1. The polypeptides may disrupt PD-L2, RGMb, PD-1, or PD-L1. The polypeptides may disrupt the interaction between PD-L2 and RGMb or disrupt the interaction between PD-1 and PD-L1.

In some embodiments, the polypeptides and proteins described herein can be isolated from cells or tissue sources by an appropriate purification scheme using standard protein purification techniques. In another embodiment, polypeptides and proteins described herein are produced by recombinant DNA techniques. Alternatively, polypeptides described herein can be chemically synthesized using standard peptide synthesis techniques.

In some embodiments, provided herein are chimeric or fusion proteins. As used herein, a "chimeric protein" or "fusion protein" comprises a polypeptide or protein described herein linked to a distinct polypeptide to which it is not linked in nature. For example, the distinct polypeptide can be fused to the N-terminus or C-terminus of the polypeptide either directly, through a peptide bond, or indirectly through a chemical linker. In some embodiments, the peptide described herein is linked to an immunoglobulin constant domain *(e.g.,* an IgG constant domain, such as a human IgG constant domain).

A chimeric or fusion polypeptide described herein can be produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, Ausubel et al., eds., John Wiley & Sons: 1992). Moreover, many expression vectors are commercially available that already encode a fusion moiety.

The polypeptides and proteins described herein can be produced in prokaryotic or eukaryotic host cells by expression of polynucleotides encoding a polypeptide(s) described herein. Alternatively, such peptides can be synthesized by chemical methods. Methods for expression of heterologous polypeptides in recombinant hosts, chemical synthesis of polypeptides, and *in vitro* translation are well known in the art and are described further in Maniatis et al., Molecular Cloning: A Laboratory Manual (1989), 2nd Ed., Cold Spring Harbor, N. Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91:501; Chaiken I. M. (1981) CRC Crit. Rev. Biochem. 11:255; Kaiser et al. (1989) Science 243:187; Merrifield, B. (1986) Science 232:342; Kent, S. B. H. (1988) Annu. Rev. Biochem. 57:957; and Offord, R. E. (1980) Semisynthetic Proteins, Wiley Publishing, which are incorporated herein by reference.

### Small Molecule Agents

Certain embodiments disclosed herein relate to agents and methods for treating or preventing a condition (e.g., any condition, disease, disorder, or indication disclosed herein) in a subject comprising administering an agent (e.g., a small molecule) that specifically binds and disrupts PD-L2, RGMb, PD-1 or PD-L1. The agent may be a small molecule that disrupts the interaction between PD-L2/RGMb or PD-1/PD-L1.

Agents useful in the methods disclosed herein may be obtained from any available source, including systematic libraries of natural and/or synthetic compounds. Agents may also be obtained by any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, *e.g.,* Zuckermann et al., 1994, J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, 1997, Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of agents may be presented in solution (e.g., Houghten, 1992, Biotechniques 13:412-421), or on beads (Lam, 1991, Nature 354:82-84), chips (Fodor, 1993, Nature 364:555-556), bacteria and/or spores, (Ladner, USP 5,223,409), plasmids (Cull et al, 1992, Proc Natl Acad Sci USA 89:1865-1869) or on phage (Scott and Smith, 1990, Science 249:386-390; Devlin, 1990, Science 249:404-406; Cwirla et al, 1990, Proc. Natl. Acad. Sci. 87:6378-6382; Felici, 1991, J. Mol. Biol. 222:301-310; Ladner, *supra.).*

### Interfering Nucleic Acid Agents

Certain embodiments disclosed herein relate to agents and methods for treating or preventing a condition (e.g., any condition, disease, disorder, or indication disclosed herein) in a subject comprising administering an agent (e.g., a interfering nucleic acid agent) that disrupts PD-L2, RGMb, PD-1 or PD-L1. The agent may be an interfering nucleic acid agent that disrupts the interaction between PD-L2/RGMb or PD-1/PD-L1.

In certain embodiments, interfering nucleic acid molecules that selectively target a product of a gene that encodes for PD-L2 or RGMb. Interfering nucleic acids generally include a sequence of cyclic subunits, each bearing a base-pairing moiety, linked by intersubunit linkages that allow the base-pairing moieties to hybridize to a target sequence in a nucleic acid (typically an RNA) by Watson-Crick base pairing, to form a nucleic acid:oligomer heteroduplex within the target sequence. Interfering RNA molecules include, but are not limited to, antisense molecules, siRNA molecules, single-stranded siRNA molecules, miRNA molecules and shRNA molecules.

Typically at least 17, 18, 19, 20, 21, 22 or 23 nucleotides of the complement of the target mRNA sequence are sufficient to mediate inhibition of a target transcript. Perfect complementarity is not necessary. In some embodiments, the interfering nucleic acid molecule is double-stranded RNA. The double-stranded RNA molecule may have a 2 nucleotide 3' overhang. In some embodiments, the two RNA strands are connected via a hairpin structure, forming a shRNA molecule. shRNA molecules can contain hairpins derived from microRNA molecules. For example, an RNAi vector can be constructed by cloning the interfering RNA sequence into a pCAG-miR30 construct containing the hairpin from the miR30 miRNA. RNA interference molecules may include DNA residues, as well as RNA residues.

Interfering nucleic acid molecules provided herein can contain RNA bases, non-RNA bases or a mixture of RNA bases and non-RNA bases. For example, interfering nucleic acid molecules provided herein can be primarily composed of RNA bases but also contain DNA bases or non-naturally occurring nucleotides.

The interfering nucleic acids can employ a variety of oligonucleotide chemistries. Examples of oligonucleotide chemistries include, without limitation, peptide nucleic acid (PNA), linked nucleic acid (LNA), phosphorothioate, 2'O-Me-modified oligonucleotides, and morpholino chemistries, including combinations of any of the foregoing. In general, PNA and LNA chemistries can utilize shorter targeting sequences because of their relatively high target binding strength relative to 2'O-Me oligonucleotides. Phosphorothioate and 2'O-Me-modified chemistries are often combined to generate 2'O-Me-modified oligonucleotides having a phosphorothioate backbone. See, *e.g.,* PCT Publication Nos. WO/2013/112053 and WO/2009/008725, incorporated by reference in their entireties.

Peptide nucleic acids (PNAs) are analogs of DNA in which the backbone is structurally homomorphous with a deoxyribose backbone, consisting of N-(2-aminoethyl) glycine units to which pyrimidine or purine bases are attached. PNAs containing natural pyrimidine and purine bases hybridize to complementary oligonucleotides obeying Watson-Crick base-pairing rules, and mimic DNA in terms of base pair recognition (Egholm, Buchardt et al. 1993). The backbone of PNAs is formed by peptide bonds rather than phosphodiester bonds, making them well-suited for antisense applications (see structure below). The backbone is uncharged, resulting in PNA/DNA or PNA/RNA duplexes that exhibit greater than normal thermal stability. PNAs are not recognized by nucleases or proteases.

Despite a radical structural change to the natural structure, PNAs are capable of sequence-specific binding in a helix form to DNA or RNA. Characteristics of PNAs include a high binding affinity to complementary DNA or RNA, a destabilizing effect caused by single-base mismatch, resistance to nucleases and proteases, hybridization with DNA or RNA independent of salt concentration and triplex formation with homopurine DNA. PANAGENE^{.™}. has developed its proprietary Bts PNA monomers (Bts; benzothiazole-2-sulfonyl group) and proprietary oligomerization process. The PNA oligomerization using Bts PNA monomers is composed of repetitive cycles of deprotection, coupling and capping. PNAs can be produced synthetically using any technique known in the art. See, *e.g.,* U.S. Pat. Nos. 6,969,766, 7,211,668, 7,022,851, 7,125,994, 7,145,006 and 7,179,896. See also U.S. Pat. Nos. 5,539,082; 5,714,331; and 5,719,262 for the preparation of PNAs. Further teaching of PNA compounds can be found in Nielsen et al., Science, 254:1497-1500, 1991. Each of the foregoing is incorporated by reference in its entirety.

Interfering nucleic acids may also contain "locked nucleic acid" subunits (LNAs). "LNAs" are a member of a class of modifications called bridged nucleic acid (BNA). BNA is characterized by a covalent linkage that locks the conformation of the ribose ring in a C30-endo (northern) sugar pucker. For LNA, the bridge is composed of a methylene between the 2'-O and the 4'-C positions. LNA enhances backbone preorganization and base stacking to increase hybridization and thermal stability.

The structures of LNAs can be found, for example, in Wengel, et al., Chemical Communications (1998) 455; Tetrahedron (1998) 54:3607, and Accounts of Chem. Research (1999) 32:301); Obika, et al., Tetrahedron Letters (1997) 38:8735; (1998) 39:5401, and Bioorganic Medicinal Chemistry (2008) 16:9230. Compounds provided herein may incorporate one or more LNAs; in some cases, the compounds may be entirely composed of LNAs. Methods for the synthesis of individual LNA nucleoside subunits and their incorporation into oligonucleotides are described, for example, in U.S. Pat. Nos. 7,572,582, 7,569,575, 7,084,125, 7,060,809, 7,053,207, 7,034,133, 6,794,499, and 6,670,461, each of which is incorporated by reference in its entirety. Typical intersubunit linkers include phosphodiester and phosphorothioate moieties; alternatively, non-phosphorous containing linkers may be employed. One embodiment is an LNA containing compound where each LNA subunit is separated by a DNA subunit. Certain compounds are composed of alternating LNA and DNA subunits where the intersubunit linker is phosphorothioate.

"Phosphorothioates" (or S-oligos) are a variant of normal DNA in which one of the nonbridging oxygens is replaced by a sulfur. The sulfurization of the internucleotide bond reduces the action of endo-and exonucleases including 5' to 3' and 3' to 5' DNA POL 1 exonuclease, nucleases S1 and P1, RNases, serum nucleases and snake venom phosphodiesterase. Phosphorothioates are made by two principal routes: by the action of a solution of elemental sulfur in carbon disulfide on a hydrogen phosphonate, or by the method of sulfurizing phosphite triesters with either tetraethylthiuram disulfide (TETD) or 3H-1, 2-bensodithiol-3-one 1, 1-dioxide (BDTD) (see, *e.g.,* Iyer et al., J. Org. Chem. 55, 4693-4699, 1990). The latter methods avoid the problem of elemental sulfur's insolubility in most organic solvents and the toxicity of carbon disulfide. The TETD and BDTD methods also yield higher purity phosphorothioates.

"2'O-Me oligonucleotides" molecules carry a methyl group at the 2'-OH residue of the ribose molecule. 2'-O-Me-RNAs show the same (or similar) behavior as DNA, but are protected against nuclease degradation. 2'-O-Me-RNAs can also be combined with phosphothioate oligonucleotides (PTOs) for further stabilization. 2'O-Me oligonucleotides (phosphodiester or phosphothioate) can be synthesized according to routine techniques in the art (see, *e.g.,* Yoo et al., Nucleic Acids Res. 32:2008-16, 2004).

The interfering nucleic acids described herein may be contacted with a cell or administered to an organism (e.g., a human). Alternatively, constructs and/or vectors encoding the interfering RNA molecules may be contacted with or introduced into a cell or organism. In certain embodiments, a viral, retroviral or lentiviral vector is used. In some embodiments the vector is an adeno-associated virus.

Typically at least 17, 18, 19, 20, 21, 22 or 23 nucleotides of the complement of the target mRNA sequence are sufficient to mediate inhibition of a target transcript. Perfect complementarity is not necessary. In some embodiments, the interfering nucleic acids contain a 1, 2 or 3 nucleotide mismatch with the target sequence. The interfering nucleic acid molecule may have a 2 nucleotide 3' overhang. If the interfering nucleic acid molecule is expressed in a cell from a construct, for example from a hairpin molecule or from an inverted repeat of the desired sequence, then the endogenous cellular machinery will create the overhangs. shRNA molecules can contain hairpins derived from microRNA molecules. For example, an RNAi vector can be constructed by cloning the interfering RNA sequence into a pCAG-miR30 construct containing the hairpin from the miR30 miRNA. RNA interference molecules may include DNA residues, as well as RNA residues.

In some embodiments, the interfering nucleic acid molecule is a siRNA molecule. Such siRNA molecules should include a region of sufficient homology to the target region, and be of sufficient length in terms of nucleotides, such that the siRNA molecule downregulate target RNA. The term "ribonucleotide" or "nucleotide" can, in the case of a modified RNA or nucleotide surrogate, also refer to a modified nucleotide, or surrogate replacement moiety at one or more positions. It is not necessary that there be perfect complementarity between the siRNA molecule and the target, but the correspondence must be sufficient to enable the siRNA molecule to direct sequence-specific silencing, such as by RNAi cleavage of the target RNA. In some embodiments, the sense strand need only be sufficiently complementary with the antisense strand to maintain the overall double-strand character of the molecule.

In addition, an siRNA molecule may be modified or include nucleoside surrogates. Single stranded regions of an siRNA molecule may be modified or include nucleoside surrogates, *e.g.,* the unpaired region or regions of a hairpin structure, *e.g.,* a region which links two complementary regions, can have modifications or nucleoside surrogates. Modification to stabilize one or more 3'- or 5'-terminus of an siRNA molecule, e.g., against exonucleases, or to favor the antisense siRNA agent to enter into RISC are also useful. Modifications can include C3 (or C6, C7, C12) amino linkers, thiol linkers, carboxyl linkers, non-nucleotidic spacers (C3, C6, C9, C12, abasic, triethylene glycol, hexaethylene glycol), special biotin or fluorescein reagents that come as phosphoramidites and that have another DMT-protected hydroxyl group, allowing multiple couplings during RNA synthesis.

Each strand of an siRNA molecule can be equal to or less than 35, 30, 25, 24, 23, 22, 21, or 20 nucleotides in length. In some embodiments, the strand is at least 19 nucleotides in length. For example, each strand can be between 21 and 25 nucleotides in length. In some embodiments, siRNA agents have a duplex region of 17, 18, 19, 29, 21, 22, 23, 24, or 25 nucleotide pairs, and one or more overhangs, such as one or two 3' overhangs, of 2-3 nucleotides.

A "small hairpin RNA" or "short hairpin RNA" or "shRNA" includes a short RNA sequence that makes a tight hairpin turn that can be used to silence gene expression via RNA interference. The shRNAs provided herein may be chemically synthesized or transcribed from a transcriptional cassette in a DNA plasmid. The shRNA hairpin structure is cleaved by the cellular machinery into siRNA, which is then bound to the RNA-induced silencing complex (RISC).

In some embodiments, shRNAs are about 15-60, 15-50, or 15-40 (duplex) nucleotides in length, about 15-30, 15-25, or 19-25 (duplex) nucleotides in length, or are about 20-24, 21-22, or 21-23 (duplex) nucleotides in length *(e.g.,* each complementary sequence of the double-stranded shRNA is 15-60, 15-50, 15-40, 15-30, 15-25, or 19-25 nucleotides in length, or about 20-24, 21-22, or 21-23 nucleotides in length, and the double-stranded shRNA is about 15-60, 15-50, 15-40, 15-30, 15-25, or 19-25 base pairs in length, or about 18-22, 19-20, or 19-21 base pairs in length). shRNA duplexes may comprise 3' overhangs of about 1 to about 4 nucleotides or about 2 to about 3 nucleotides on the antisense strand and/or 5'-phosphate termini on the sense strand. In some embodiments, the shRNA comprises a sense strand and/or antisense strand sequence of from about 15 to about 60 nucleotides in length (e.g., about 15-60, 15-55, 15-50, 15-45, 15-40, 15-35, 15-30, or 15-25 nucleotides in length), or from about 19 to about 40 nucleotides in length (e.g., about 19-40, 19-35, 19-30, or 19-25 nucleotides in length), or from about 19 to about 23 nucleotides in length *(e.g.,* 19, 20, 21, 22, or 23 nucleotides in length).

Non-limiting examples of shRNA include a double-stranded polynucleotide molecule assembled from a single-stranded molecule, where the sense and antisense regions are linked by a nucleic acid-based or non-nucleic acid-based linker; and a double-stranded polynucleotide molecule with a hairpin secondary structure having self-complementary sense and antisense regions. In some embodiments, the sense and antisense strands of the shRNA are linked by a loop structure comprising from about 1 to about 25 nucleotides, from about 2 to about 20 nucleotides, from about 4 to about 15 nucleotides, from about 5 to about 12 nucleotides, or 1, 2, 3, 4, 5, 6, 7, 8, 9, 10, 11, 12, 13, 14, 15, 16, 17, 18, 19, 20, 21, 22, 23, 24, 25, or more nucleotides.

Additional embodiments related to the shRNAs, as well as methods of designing and synthesizing such shRNAs, are described in U.S. patent application publication number 2011/0071208, the disclosure of which is herein incorporated by reference in its entirety for all purposes.

In some embodiments, provided herein are micro RNAs (miRNAs). miRNAs represent a large group of small RNAs produced naturally in organisms, some of which regulate the expression of target genes. miRNAs are formed from an approximately 70 nucleotide single-stranded hairpin precursor transcript by Dicer. miRNAs are not translated into proteins, but instead bind to specific messenger RNAs, thereby blocking translation. In some instances, miRNAs base-pair imprecisely with their targets to inhibit translation.

In some embodiments, antisense oligonucleotide compounds are provided herein. In certain embodiments, the degree of complementarity between the target sequence and antisense targeting sequence is sufficient to form a stable duplex. The region of complementarity of the antisense oligonucleotides with the target RNA sequence may be as short as 8-11 bases, but can be 12-15 bases or more, *e.g.,* 10-40 bases, 12-30 bases, 12-25 bases, 15-25 bases, 12-20 bases, or 15-20 bases, including all integers in between these ranges. An antisense oligonucleotide of about 14-15 bases is generally long enough to have a unique complementary sequence.

In certain embodiments, antisense oligonucleotides may be 100% complementary to the target sequence, or may include mismatches, e.g., to improve selective targeting of allele containing the disease-associated mutation, as long as a heteroduplex formed between the oligonucleotide and target sequence is sufficiently stable to withstand the action of cellular nucleases and other modes of degradation which may occur *in vivo.* Hence, certain oligonucleotides may have about or at least about 70% sequence complementarity, e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence complementarity, between the oligonucleotide and the target sequence. Oligonucleotide backbones that are less susceptible to cleavage by nucleases are discussed herein. Mismatches, if present, are typically less destabilizing toward the end regions of the hybrid duplex than in the middle. The number of mismatches allowed will depend on the length of the oligonucleotide, the percentage of G:C base pairs in the duplex, and the position of the mismatch(es) in the duplex, according to well understood principles of duplex stability.

Interfering nucleic acid molecules can be prepared, for example, by chemical synthesis, *in vitro* transcription, or digestion of long dsRNA by Rnase III or Dicer. These can be introduced into cells by transfection, electroporation, or other methods known in the art. See Hannon, GJ, 2002, RNA Interference, Nature 418: 244-251; Bernstein E et al., 2002, The rest is silence. RNA 7: 1509-1521; Hutvagner G et al., RNAi: Nature abhors a double-strand. Curr. Opin. Genetics & Development 12: 225-232; Brummelkamp, 2002, A system for stable expression of short interfering RNAs in mammalian cells. Science 296: 550-553; Lee NS, Dohjima T, Bauer G, Li H, Li M-J, Ehsani A, Salvaterra P, and Rossi J. (2002). Expression of small interfering RNAs targeted against HIV-1 rev transcripts in human cells. Nature Biotechnol. 20:500-505; Miyagishi M, and Taira K. (2002). U6-promoter-driven siRNAs with four uridine 3' overhangs efficiently suppress targeted gene expression in mammalian cells. Nature Biotechnol. 20:497-500; Paddison PJ, Caudy AA, Bernstein E, Hannon GJ, and Conklin DS. (2002). Short hairpin RNAs (shRNAs) induce sequence-specific silencing in mammalian cells. Genes & Dev. 16:948-958; Paul CP, Good PD, Winer I, and Engelke DR. (2002). Effective expression of small interfering RNA in human cells. Nature Biotechnol. 20:505-508; Sui G, Soohoo C, Affar E-B, Gay F, Shi Y, Forrester WC, and Shi Y. (2002). A DNA vector-based RNAi technology to suppress gene expression in mammalian cells. Proc. Natl. Acad. Sci. USA 99(6):5515-5520; Yu J-Y, DeRuiter SL, and Turner DL. (2002). RNA interference by expression of short-interfering RNAs and hairpin RNAs in mammalian cells. Proc. Natl. Acad. Sci. USA 99(9):6047-6052.

In the present methods, an interfering nucleic acid molecule or an interfering nucleic acid encoding polynucleotide can be administered to the subject, for example, as naked nucleic acid, in combination with a delivery reagent, and/or as a nucleic acid comprising sequences that express an interfering nucleic acid molecule. In some embodiments the nucleic acid comprising sequences that express the interfering nucleic acid molecules are delivered within vectors, e.g. plasmid, viral and bacterial vectors. Any nucleic acid delivery method known in the art can be used in the methods described herein. Suitable delivery reagents include, but are not limited to, *e.g.,* the Mirus Transit TKO lipophilic reagent; lipofectin; lipofectamine; cellfectin; polycations (e.g., polylysine), atelocollagen, nanoplexes and liposomes. The use of atelocollagen as a delivery vehicle for nucleic acid molecules is described in Minakuchi et al. Nucleic Acids Res., 32(13):e109 (2004); Hanai et al. Ann NY Acad Sci., 1082:9-17 (2006); and Kawata et al. Mol Cancer Ther., 7(9):2904-12 (2008); each of which is incorporated herein in their entirety. Exemplary interfering nucleic acid delivery systems are provided in U.S. Patent Nos. 8,283,461, 8,313,772, 8,501,930. 8,426,554, 8,268,798 and 8,324,366, each of which is hereby incorporated by reference in its entirety.

In some embodiments of the methods described herein, liposomes are used to deliver an inhibitory oligonucleotide to a subject. Liposomes suitable for use in the methods described herein can be formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example, as described in Szoka et al. (1980), Ann. Rev. Biophys. Bioeng. 9:467; and U.S. Pat. Nos. 4,235,871, 4,501,728, 4,837,028, and 5,019,369, the entire disclosures of which are herein incorporated by reference.

The liposomes for use in the present methods can also be modified so as to avoid clearance by the mononuclear macrophage system ("MMS") and reticuloendothelial system ("RES"). Such modified liposomes have opsonization-inhibition moieties on the surface or incorporated into the liposome structure.

Opsonization-inhibiting moieties for use in preparing the liposomes described herein are typically large hydrophilic polymers that are bound to the liposome membrane. As used herein, an opsonization inhibiting moiety is "bound" to a liposome membrane when it is chemically or physically attached to the membrane, e.g., by the intercalation of a lipid-soluble anchor into the membrane itself, or by binding directly to active groups of membrane lipids. These opsonization-inhibiting hydrophilic polymers form a protective surface layer that significantly decreases the uptake of the liposomes by the MMS and RES; *e.g.,* as described in U.S. Pat. No. 4,920,016, the entire disclosure of which is herein incorporated by reference.

In some embodiments, opsonization inhibiting moieties suitable for modifying liposomes are water-soluble polymers with a number-average molecular weight from about 500 to about 40,000 daltons, or from about 2,000 to about 20,000 daltons. Such polymers include polyethylene glycol (PEG) or polypropylene glycol (PPG) derivatives; e.g., methoxy PEG or PPG, and PEG or PPG stearate; synthetic polymers such as polyacrylamide or poly N-vinyl pyrrolidone; linear, branched, or dendrimeric polyamidoamines; polyacrylic acids; polyalcohols, e.g., polyvinylalcohol and polyxylitol to which carboxylic or amino groups are chemically linked, as well as gangliosides, such as ganglioside GM1. Copolymers of PEG, methoxy PEG, or methoxy PPG, or derivatives thereof, are also suitable. In addition, the opsonization inhibiting polymer can be a block copolymer of PEG and either a polyamino acid, polysaccharide, polyamidoamine, polyethyleneamine, or polynucleotide. The opsonization inhibiting polymers can also be natural polysaccharides containing amino acids or carboxylic acids, e.g., galacturonic acid, glucuronic acid, mannuronic acid, hyaluronic acid, pectic acid, neuraminic acid, alginic acid, carrageenan; aminated polysaccharides or oligosaccharides (linear or branched); or carboxylated polysaccharides or oligosaccharides, *e.g.,* reacted with derivatives of carbonic acids with resultant linking of carboxylic groups. In some embodiments, the opsonization-inhibiting moiety is a PEG, PPG, or derivatives thereof. Liposomes modified with PEG or PEG-derivatives are sometimes called "PEGylated liposomes."

### CRISPR/Gene Editing

Certain embodiments disclosed herein relate to agents and methods for treating or preventing a condition (e.g., any condition, disease, disorder, or indication disclosed herein) in a subject comprising administering an agent (e.g., a gene editing agent) that disrupts PD-L2, RGMb, PD-1 or PD-L1. The agent may be a gene editing agent that disrupts the interaction between PD-L2/RGMb or PD-1/PD-L1.

In some embodiments, the agent disclosed herein is an agent for genome editing (e.g., an agent used to delete at least a portion of a gene that encodes a PD-L2 or RGMb peptide). Deletion of DNA may be performed using gene therapy to knock-out or disrupt the target gene. As used herein, a "knock-out" can be a gene knock-down or the gene can be knocked out by a mutation such as, a point mutation, an insertion, a deletion, a frameshift, or a missense mutation by techniques known in the art, including, but not limited to, retroviral gene transfer. In some embodiments, the agent is a nuclease (e.g., a zinc finger nuclease or a TALEN). Zinc-finger nucleases (ZFNs) are artificial restriction enzymes generated by fusing a zinc finger DNA-binding domain to a DNA-cleavage domain. Zinc finger domains can be engineered to target desired DNA sequences, which enable zinc-finger nucleases to target unique sequence within a complex genome. By taking advantage of endogenous DNA repair machinery, these reagents can be used to precisely alter the genomes of higher organisms. Other technologies for genome customization that can be used to knock out genes are meganucleases and TAL effector nucleases (TALENs). A TALEN is composed of a TALE DNA binding domain for sequence-specific recognition fused to the catalytic domain of an endonuclease that introduces double-strand breaks (DSB). The DNA binding domain of a TALEN is capable of targeting with high precision a large recognition site (for instance, 17 bp). Meganucleases are sequence-specific endonucleases, naturally occurring "DNA scissors," originating from a variety of single-celled organisms such as bacteria, yeast, algae and some plant organelles. Meganucleases have long recognition sites of between 12 and 30 base pairs. The recognition site of natural meganucleases can be modified in order to target native genomic DNA sequences (such as endogenous genes).

In another embodiment, the agent comprises a CRISPR-Cas9 guided nuclease and/or a sgRNA (Wiedenheft et al., "RNA-Guided Genetic Silencing Systems in Bacteria and Archaea," Nature 482:331-338 (2012); Zhang et al., "Multiplex Genome Engineering Using CRISPR/Cas Systems," Science 339(6121): 819-23 (2013); and Gaj et al., "ZFN, TALEN, and CRISPR/Cas-based Methods for Genome Engineering," Cell 31(7):397-405 (2013), which are hereby incorporated by reference in their entirety). Like the TALENs and ZFNs, CRISPR-Cas9 interference is a genetic technique which allows for sequence-specific control of gene expression in prokaryotic and eukaryotic cells by guided nuclease double-stranded DNA cleavage. It is based on the bacterial immune system - derived CRISPR (clustered regularly interspaced palindromic repeats) pathway. In some embodiments, the agent is an sgRNA. An sgRNA combines tracrRNA and crRNA, which are separate molecules in the native CRISPR/Cas9 system, into a single RNA construct, simplifying the components needed to use CRISPR/Cas9 for genome editing. In some embodiments, the crRNA of the sgRNA has complementarity to at least a portion of a gene that encodes PD-L2 or RGMb (or a fragment thereof). In some embodiments, the sgRNA may target at least a portion of a gene that encodes a PD-L2 or RGMb protein.

### Pharmaceutical Compositions

In certain embodiments, provided herein is a composition, e.g., a pharmaceutical composition, containing at least one agent described herein together with a pharmaceutically acceptable carrier. In one embodiment, the composition includes a combination of multiple *(e.g.,* two or more) agents described herein.

In some embodiments, the composition comprises an agent that disrupts the PD-L2/RGMb interaction, e.g., and antibody. Exemplary antibodies can be found in patents and published applications such as: US Patent No. 9,845,356, US Patent No. 10,370,448, US Patent Publication 2018/0002422, WO Pat. Publication WO2002000730, and US Patent Publication 2018/0258171, hereby incorporated by reference in their entireties.

In some embodiments, the composition comprises an agent that disrupts PD-L1 or PD-1, e.g., cemiplimab (REGN2810), nivolumab (BMS-936558, MDX-1106, ONO-4538), pembrolizumab (MK-3475, SCH 900475), SHR1210, sintilimab (IBI308), spartalizumab (PDR001), tislelizumab (BGB-A317), pidilizumab, BCD-100, toripalimab (JS001), PF-06801591, AB122, AK105, AMG 404, BCD-100, BI 754091, F520, HLX10, HX008, JTX-4014, LZM009, MEDI0680, MGA012, Sym021, TSR-042, PSB205, MGD019, MGD013, AK104, XmAb20717, RO7121661, CX-188, atezolizumab (MPDL3280A, RG7446, RO5541267), durvalumab (MEDI4736, MEDI-4736), avelumab (MSB0010718C), FS118, BCD-135, BGB-A333, CBT-502, CK-301, CS1001, FAZ053, HLX20, KN035, MDX-1105, MSB2311, SHR-1316, TG-1501, ZKAB001, INBRX-105, MCLA-145, KN046, M7824, or LY3415244.

As described in detail below, the pharmaceutical compositions disclosed herein may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; or (2) parenteral administration, for example, by subcutaneous, intramuscular, intravenous, intrathecal, intracerebral or epidural injection as, for example, a sterile solution or suspension, or sustained-release formulation.

Methods of preparing these formulations or compositions include the step of bringing into association an agent described herein with the carrier and, optionally, one or more accessory ingredients. In general, the formulations are prepared by uniformly and intimately bringing into association an agent described herein with liquid carriers, or finely divided solid carriers, or both, and then, if necessary, shaping the product.

Pharmaceutical compositions suitable for parenteral administration comprise one or more agents described herein in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions include water, ethanol, dimethyl sulfoxide (DMSO), polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Regardless of the route of administration selected, the agents provided herein, which may be used in a suitable hydrated form, and/or the pharmaceutical compositions disclosed herein, are formulated into pharmaceutically-acceptable dosage forms by conventional methods known to those of skill in the art.

As described in detail below, the pharmaceutical compositions and/or agents disclosed herein may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; or (2) parenteral administration, for example, by subcutaneous, intramuscular, intravenous, intrathecal, intracerebral or epidural injection as, for example, a sterile solution or suspension, or sustained-release formulation. Methods of preparing pharmaceutical formulations or compositions include the step of bringing into association an agent described herein with the carrier and, optionally, one or more accessory ingredients. In general, the formulations are prepared by uniformly and intimately bringing into association an agent described herein with liquid carriers, or finely divided solid carriers, or both, and then, if necessary, shaping the product.

Pharmaceutical compositions suitable for parenteral administration comprise one or more agents described herein in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions include water, ethanol, dimethyl sulfoxide (DMSO), polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

### Additional Methods

The agents (e.g., the first and/or second agents, or any additional agent) and/or compositions (e.g., pharmaceutical compositions) may be administered systemically or locally to the tumor present in the subject. In some embodiments, the agent or pharmaceutical composition is administered with an additional therapeutic agent. In some embodiments, the additional therapeutic agent is a chemotherapeutic agent. Exemplary chemotherapeutic agents include alkylating agents such as thiotepa and cyclophosphamide (Cytoxan^{™}); alkyl sulfonates such as busulfan, improsulfan and piposulfan; aziridines such as benzodopa, carboquone, meturedopa, and uredopa; emylerumines and memylamelamines including alfretamine, triemylenemelamine, triethylenephosphoramide, triethylenethiophosphoramide, and trimemylolomelamine; acetogenins (especially bullatacin and bullatacinone); a camptothecin (including synthetic analogue topotecan); bryostatin; callystatin; CC-1065 (including its adozelesin, carzelesin and bizelesin synthetic analogues); cryptophycins (articularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, foremustine, lomustine, nimustine, ranimustine; antibiotics such as the enediyne antibiotics (e.g., calicheamicin, especially calicheamicin gammall and calicheamicin phili); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, carrninomycin, carzinophilin, chromomycins, dactinomycin, daunorubicin, detorubicin, 6-diazo-5-oxo-L-norleucine, doxorubicin (Adramycin^{™}) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as demopterin, methotrexate, pteropterin, trimetrexate; purine analogs such as fludarabine, 6-mercaptopurine, thiamiprine, thioguanine; pyrimidine analogues such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replinisher such as frolinic acid; aceglatone; aldophosphamide glycoside; aminolevulinic acid; eniluracil; amsacrine; hestrabucil; bisantrene; edatraxate; defofamine; demecolcine; diaziquone; elformthine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{™}; razoxane; rhizoxin; sizofiran; spirogermanium; tenuazonic acid; triaziquone; 2,2',2"-tricUorotriemylamine; trichothecenes (especially T-2 toxin, verracurin A, roridin A and anguidine); urethane; vindesine; dacarbazine; mannomustine; mitobronitol; mitolactol; pipobroman; gacytosine; arabinoside ("Ara-C"); cyclophosphamide; thiopeta; taxoids, e.g., paclitaxel (Taxol^{™}, Bristol Meyers Squibb Oncology, Princeton, N.J.) and docetaxel (Taxoteret^{™}, Rhone-Poulenc Rorer, Antony, France); chlorambucil; gemcitabine (Gemzar^{™}); 6-thioguanine; mercaptopurine; methotrexate; platinum analogs such as cisplatin and carboplatin; vinblastine; platinum; etoposide (VP-16); ifosfamide; mitroxantrone; vancristine; vinorelbine (Navelbine^{™}); novantrone; teniposide; edatrexate; daunomycin; aminopterin; xeoloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included in the definition of "chemotherapeutic agent" are anti-hormonal agents that act to regulate or inhibit hormone action on tumors such as anti-estrogens and selective estrogen receptor modulators (SERMs), including, for example, tamoxifen (including Nolvadex^{™}), raloxifene, droloxifene, 4-hydroxytamoxifen, trioxifene, keoxifene, LY117018, onapristone, and toremifene (Fareston^{™}); inhibitors of the enzyme aromatase, which regulates estrogen production in the adrenal glands, such as, for example, 4(5)-imidazoles, aminoglutethimide, megestrol acetate (Megace^{™}), exemestane, formestane, fadrozole, vorozole (Rivisor^{™}), letrozole (Femara^{™}), and anastrozole (Arimidex^{™}); and anti-androgens such as flutamide, nilutamide, bicalutamide, leuprohde, and goserelin; and pharmaceutically acceptable salts, acids or derivatives of any of the above. In some embodiments, the additional therapeutic agent is an immune checkpoint inhibitor. Immune Checkpoint inhibition broadly refers to inhibiting the checkpoints that cancer cells can produce to prevent or downregulate an immune response. Examples of immune checkpoint proteins are CTLA-4, PD-1, VISTA, B7-H2, B7-H3, PD-L1, B7-H4, B7-H6, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, HHLA2, butyrophilins, A2aR, and combinations thereof.

### Indications

In some aspects, provided herein are methods of treating a cancer by administering to a subject (e.g., to a tumor present in a subject or to the subject orally) the agents (e.g., the first and/or second agents, or any additional agent) described herein.

In some embodiments, the methods described herein may be used to treat any cancerous or pre-cancerous tumor. In some embodiments, the cancer includes a solid tumor. Cancers that may be treated by methods and compositions provided herein include, but are not limited to, cancer cells from the bladder, blood, bone, bone marrow, brain, breast (e.g., estrogen receptor (ER)-positive breast cancer, triple negative breast cancer, or HER2 positive breast cancer), colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometrioid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; mammary paget's disease; acinar cell carcinoma; adenosquamous carcinoma; adenocarcinoma w/squamous metaplasia; malignant thymoma; malignant ovarian stromal tumor; malignant thecoma; malignant granulosa cell tumor; and malignant roblastoma; sertoli cell carcinoma; malignant leydig cell tumor; malignant lipid cell tumor; malignant paraganglioma; malignant extra-mammary paraganglioma; pheochromocytoma; glomangiosarcoma; malignant melanoma; amelanotic melanoma; superficial spreading melanoma; malignant melanoma in giant pigmented nevus; epithelioid cell melanoma; malignant blue nevus; sarcoma; fibrosarcoma; malignant fibrous histiocytoma; myxosarcoma; liposarcoma; leiomyosarcoma; rhabdomyosarcoma; embryonal rhabdomyosarcoma; alveolar rhabdomyosarcoma; stromal sarcoma; malignant mixed tumor; mullerian mixed tumor; nephroblastoma; hepatoblastoma; carcinosarcoma; malignant mesenchymoma; malignant brenner tumor; malignant phyllodes tumor; synovial sarcoma; malignant mesothelioma; dysgerminoma; embryonal carcinoma; malignant teratoma; malignant struma ovarii; choriocarcinoma; malignant mesonephroma; hemangiosarcoma; malignant hemangioendothelioma; kaposi's sarcoma; malignant hemangiopericytoma; lymphangiosarcoma; osteosarcoma; juxtacortical osteosarcoma; chondrosarcoma; malignant chondroblastoma; mesenchymal chondrosarcoma; giant cell tumor of bone; ewing's sarcoma; malignant odontogenic tumor; ameloblastic odontosarcoma; malignant ameloblastoma; ameloblastic fibrosarcoma; malignant pinealoma; chordoma; malignant glioma; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; malignant meningioma; neurofibrosarcoma; malignant neurilemmoma; malignant granular cell tumor; malignant lymphoma; Hodgkin's disease; Hodgkin's lymphoma; paragranuloma; small lymphocytic malignant lymphoma; diffuse large cell malignant lymphoma; follicular malignant lymphoma; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

In some embodiments, the subject has cancer. In some embodiments, the cancer comprises a solid tumor. In some embodiments, the tumor is an adenocarcinoma, an adrenal tumor, an anal tumor, a bile duct tumor, a bladder tumor, a bone tumor, a blood born tumor, a brain/CNS tumor, a breast tumor, a cervical tumor, a colorectal tumor, an endometrial tumor, an esophageal tumor, an Ewing tumor, an eye tumor, a gallbladder tumor, a gastrointestinal, a kidney tumor, a laryngeal or hypopharyngeal tumor, a liver tumor, a lung tumor, a mesothelioma tumor, a multiple myeloma tumor, a muscle tumor, a nasopharyngeal tumor, a neuroblastoma, an oral tumor, an osteosarcoma, an ovarian tumor, a pancreatic tumor, a penile tumor, a pituitary tumor, a primary tumor, a prostate tumor, a retinoblastoma, a Rhabdomyosarcoma, a salivary gland tumor, a soft tissue sarcoma, a melanoma, a metastatic tumor, a basal cell carcinoma, a Merkel cell tumor, a testicular tumor, a thymus tumor, a thyroid tumor, a uterine tumor, a vaginal tumor, a vulvar tumor, or a Wilms tumor.

### EXEMPLIFICATION

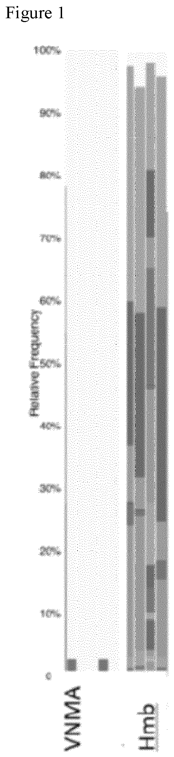

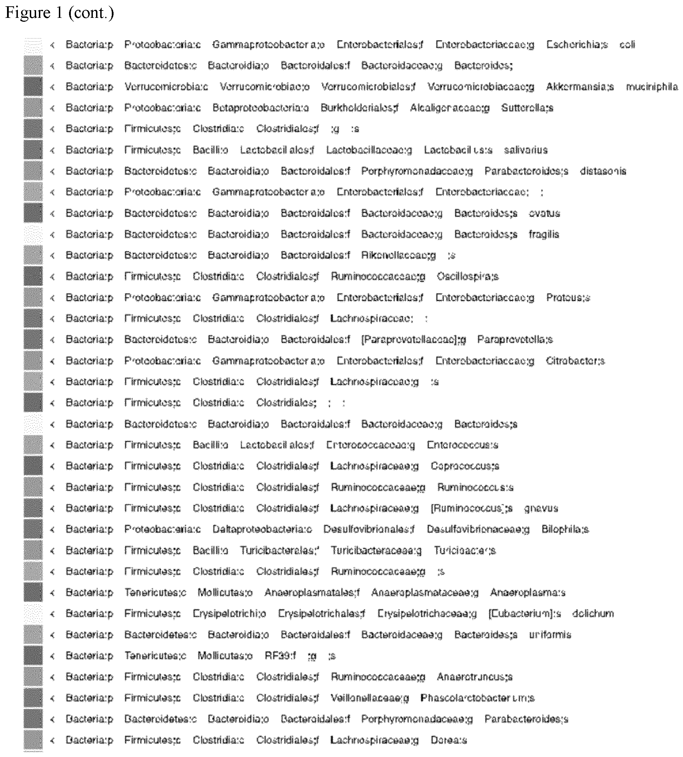

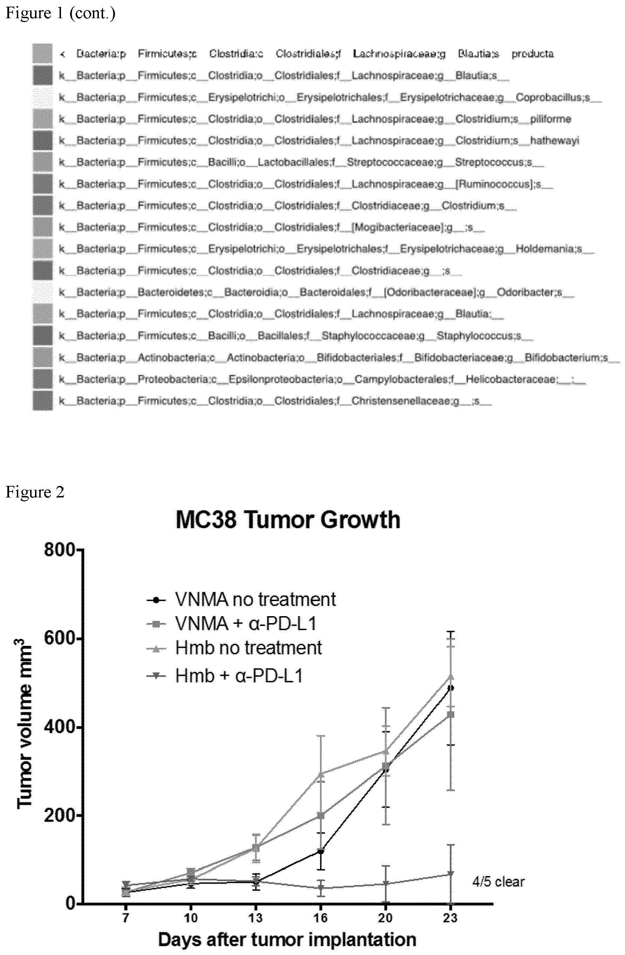

Figures 1-4 are data plots showing that mice treated with broad spectrum antibiotics (Vancomycin, Neomycin, Metronidazole, and Ampicillin or "VNMA") have dysbiosis, an unhealthy microbiota.

Figure 1 shows that mice treated with broad spectrum antibiotics (VNMA) for 17 days have an altered microbiota, referred to as dysbiosis. This microbiota is made up dominantly of a Proteobacteria, E. coli. Mice given a dose of healthy human microbiota (Hmb) have a much more diverse microbiota.

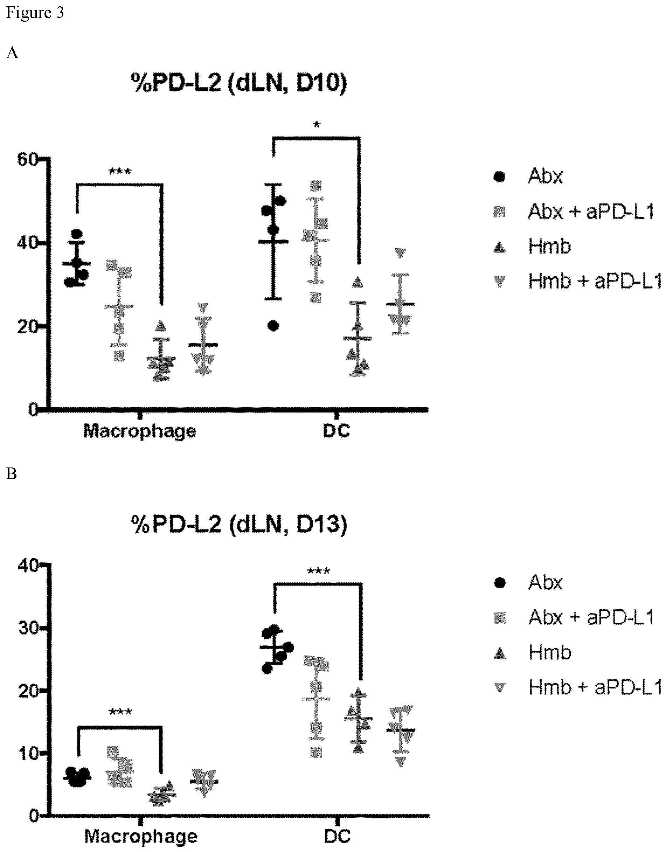

Figure 2 shows that mice given VNMA do not respond to anti-PD-Ll therapy whereas mice given an oral dose of Hmb can clear or have significantly smaller tumors indicating that dysbiosis inhibits the anti-tumor effect of anti-PD-Ll therapy. To determine how dysbiosis abrogates the anti-tumor effects of anti-PD-Ll therapy, the expression of surface markers on the immune cells in the tumor draining lymph nodes of VNMA versus Hmb mice were compared. It was found that PD-L2 expression was significantly lower in macrophages and dendritic cells of Hmb mice, but higher in VNMA mice (Figure 3).

To determine if disrupting PD-L2 in VNMA mice would reverse the effects of dysbiosis on anti-tumor immunity, anti-PD-Ll and anti PD-L2 therapy was combined in VNMA treated mice. Figure 4 shows that while anti-PD-Ll and anti-PD-L2 individually do not promote an anti-tumor response in VNMA treated mice, they synergistically promote an anti-tumor response. These data show that combined anti-PD-Ll and anti-PD-L2 therapy can reverse the effects of dysbiosis and promote anti-tumor responses. These data imply that in the clinic, patients with dysbiosis and poor response to anti-PD-Ll treatment, could benefit from combined anti-PD-Ll and anti-PD-L2 therapy.

Figures 5 shows the experimental timeline for Figures 1-4. Mice are given antibiotics (0.5 mg/ml Vancomycin, 1 mg/ml Neomycin, 1mg/ml metronidazole, 1mg/ml Ampicillin) in drinking water 4 days before tumor implantation. On day zero, 2.5 × 10⁵ MC38 tumor cells are implanted subcutaneously in the abdomen of 6 week old female mice. On days 7, 10, 13, 16 mice are treated with 100 µg of Isotype or anti-PD-Ll by intraperitoneal injection. On day 7, half of the mice are orally gavaged with a slurry of Hmb feces and antibiotics are removed from the drinking water; the other half of the mice do not receive Hmb feces and continue with antibiotics in the drinking water for the remainder of the experiment. Tumors are measured on days 7, 10, 13, 16, 20, 23.

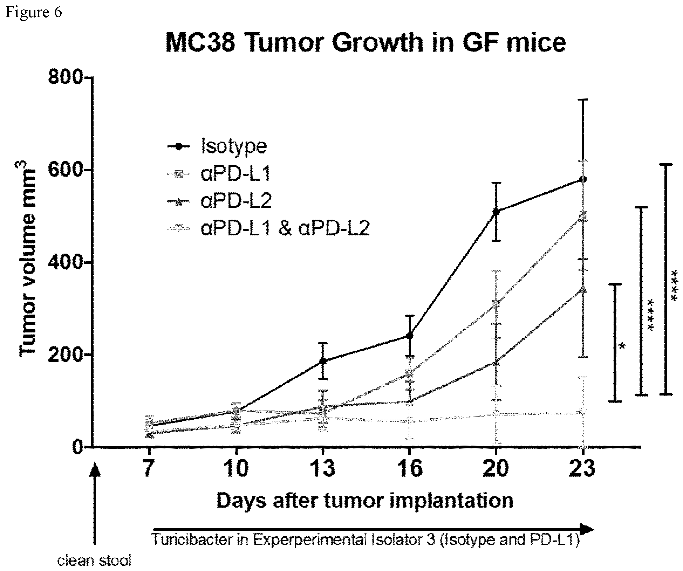

Figures 6-9 show that in germ free mice, anti-PD-1 or anti-PD-Ll therapy alone does not promote anti-tumor response, but synergistically promotes an anti-tumor response if combined with anti-PD-L2 or anti-RGMb. 2.5 ×10⁵ MC38 tumor cells were implanted subcutaneously in the abdomens of 6 week old female germ free mice in germ free isolators. Antibody treatments were administered by intraperitoneal injections on days 7, 10, 13, 16. All reagents and supplies were sterilized and mice were maintained in sterile isolators throughout the experiments.

Figure 6 shows that combined anti-PD-Ll and anti-PD-L2 therapy, but not anti-PD-L1 or anti-PD-L2 therapy, promotes an anti-tumor response in germ free mice.

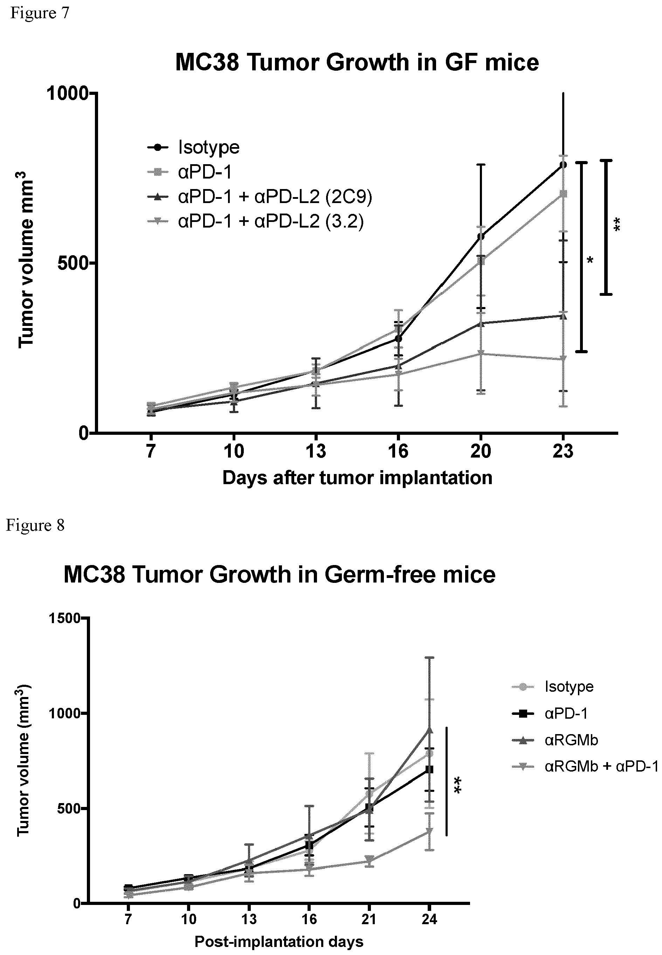

Figure 7 shows that combined anti-PD-1 and anti-PD-L2 therapy, but not anti-PD-1 or anti-PD-L2 therapy, promotes an anti-tumor response in germ free mice.

In particular, Figures 6 and 7 show that anti-PD-1 therapy does not promote an anti-tumor response in GF mice, but anti-PD-1 therapy combined with antibodies targeting either PD-L2/PD-1 and PD-L2/RGMb (mAb clone 3.2) or only PD-L2/RGMb (mAb clone GF17.2C9) can promote an anti-tumor response. These data suggest that targeting PD-L2 mediated signaling pathways including the PD-L2/RGMb pathway is important for anti-tumor responses in patients receiving anti-PD-1 therapy.

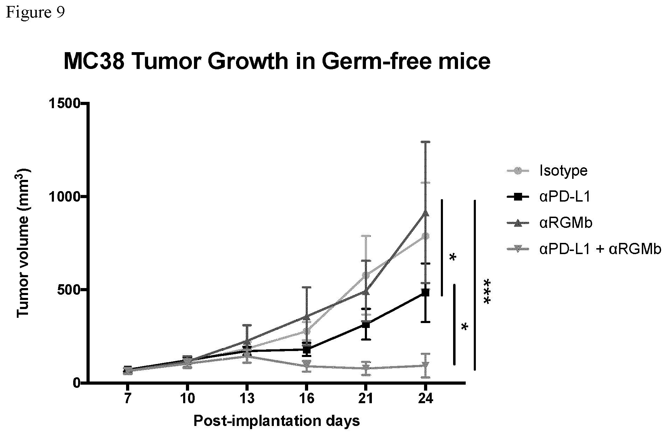

Because anti-PD-L2 mAb clone GF17. 2C9 (2C9), which disrupts the interaction between PD-L2 and RGMb without disrupting the interaction between PD-L2 and PD-1, promotes an anti-tumor response in GF mice with either anti-PD-Ll or anti-PD-1 treatment, we tested whether targeting RGMb instead of PD-L2 could also promote an anti-tumor response in GF mice treated with either anti-PD-1 or anti-PD-L1. It was found that combination anti-RGMb and either anti-PD-1 (Figure 8) or anti-PD-Ll (Figure 9) promotes an anti-tumor response in GF mice suggesting that disrupting the PD-L2/RGMb response by either targeting PD-L2 or targeting RGMb can promote an anti-tumor response in patients receiving anti-PD-1 or anti-PD-Ll therapy.

The conjoint therapies provided herein will be effective in a variety of tumor types and cancers. Provided herein are methods of treating or preventing melanoma in a subject, comprising administering to the subject a first agent that specifically binds to PD-L2 conjointly with a second agent that specifically binds to PD-1 or PD-L1. Also provided herein are methods of treating or preventing colon cancer in a subject, comprising administering to the subject a first agent that specifically binds to PD-L2 conjointly with a second agent that specifically binds to PD-1 or PD-L1

### Incorporation by Reference

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

### Equivalents

Those skilled in the art will recognize, or be able to ascertain using no more than routine experimentation, many equivalents to the specific embodiments are described herein. Such equivalents are intended to be encompassed by the following claims.

Further preferred embodiments are described in the following. In one preferred embodiment the disclosure relates to a method of treating or preventing cancer in a subject, comprising administering to the subject a first agent that specifically binds to PD-L2 conjointly with a second agent that specifically binds to PD-1 or PD-L1. It is preferred that the first agent is an antibody or the first agent is an antibody that blocks or disrupts PD-L2. 4, preferably a monoclonal antibody or/and a humanized antibody or/and a bispecific antibody. In a preferred embodiment the second agent is an antibody, preferably an antibody that blocks PD-1, preferably a monoclonal antibody or/and a humanized antibody or/and a bispecific antibody. In one aspect of the disclosure the antibody that blocks PD-1 is selected from cemiplimab (REGN2810), nivolumab (BMS-936558, MDX-1106, ONO-4538), pembrolizumab (MK-3475, SCH 900475), SHR1210, sintilimab (IBI308), spartalizumab (PDR001), tislelizumab (BGB-A317), pidilizumab, BCD-100, toripalimab (JS001), PF-06801591, AB122, AK105, AMG 404, BCD-100, BI 754091, F520, HLX10, HX008, JTX-4014, LZM009, MEDI0680, MGA012, Sym021, TSR-042, PSB205, MGD019, MGD013, AK104, XmAb20717, RO7121661, and CX-188. In one aspect of the disclosure the second agent is an antibody that blocks PD-L1. In one aspect of the disclosure the antibody that blocks PD-L1 is a monoclonal antibody. In one aspect of the disclosure the antibody that blocks PD-L1 is a humanized antibody, a bispecific antibody, preferably wherein the antibody that blocks PD-L1 is selected from atezolizumab (MPDL3280A, RG7446, RO5541267), durvalumab (MEDI4736, MEDI-4736), avelumab (MSB0010718C), FS118, BCD-135, BGB-A333, CBT-502, CK-301, CS1001, FAZ053, HLX20, KN035, MDX-1105, MSB2311, SHR-1316, TG-1501, ZKAB001, INBRX-105, MCLA-145, KN046, M7824, and LY3415244. In one aspect of the disclosure the subject is nonresponsive to immune checkpoint inhibitor therapy. In one aspect of the disclosure the subject is nonresponsive to anti-PD-1 therapy or anti-PD-Ll therapy. In one aspect of the disclosure at least one antibiotic was administered to the subject within a period of time prior to administration of the first agent. In one aspect of the disclosure the at least one antibiotic is Vancomycin, Neomycin, Metronidazole, or Ampicillin. In one aspect of the disclosure at least two antibiotics are administered to the patient within the period of time prior to administration of the first agent. In one aspect of the disclosure at least three antibiotics are administered to the patient within the period of time prior to administration of the first agent. In one aspect of the disclosure at least four antibiotics are administered to the patient within the period of time prior to administration of the first agent. In one aspect of the disclosure the period of time is 1 week, 3 weeks, 1 month, 3 months, or 6 months. In one aspect of the disclosure the subject has dysbiosis. In one aspect of the disclosure the gastrointestinal microbiota of the subject comprises high levels of E. coli or bacteria from phyla that is not Bacteroidetes and Firmicutes. In one aspect of the disclosure the first agent or/and the second agent is administered to the subject systemically, orally, parenterally, or intravenously. In one aspect of the disclosure the cancer is lung cancer, a breast cancer, a colon cancer, a cervical cancer, a pancreatic cancer, a renal cancer, a stomach cancer, a GI cancer, a liver cancer, a bone cancer, a hematological cancer, a neural tissue cancer, a melanoma, a thyroid cancer, a ovarian cancer, a testicular cancer, a prostate cancer, a cervical cancer, a vaginal cancer, or a bladder cancer. In one aspect of the disclosure the cancer comprises a tumor, wherein the tumor is an adenocarcinoma, an adrenal tumor, an anal tumor, a bile duct tumor, a bladder tumor, a bone tumor, a brain/CNS tumor, a breast tumor, a cervical tumor, a colorectal tumor, an endometrial tumor, an esophageal tumor, an Ewing tumor, an eye tumor, a gallbladder tumor, a gastrointestinal, a kidney tumor, a laryngeal or hypopharyngeal tumor, a liver tumor, a lung tumor, a mesothelioma tumor, a multiple myeloma tumor, a muscle tumor, a nasopharyngeal tumor, a neuroblastoma, an oral tumor, an osteosarcoma, an ovarian tumor, a pancreatic tumor, a penile tumor, a pituitary tumor, a primary tumor, a prostate tumor, a retinoblastoma, a Rhabdomyosarcoma, a salivary gland tumor, a soft tissue sarcoma, a melanoma, a metastatic tumor, a basal cell carcinoma, a Merkel cell tumor, a testicular tumor, a thymus tumor, a thyroid tumor, a uterine tumor, a vaginal tumor, a vulvar tumor, or a Wilms tumor. In one aspect of the disclosure an additional agent is administered, preferably wherein the additional agent is a chemotherapeutic agent, or wherein the additional agent is an additional immune checkpoint inhibitor, preferably wherein the additional immune checkpoint inhibitor comprises an antibody or agent specific for an immune checkpoint protein selected from CTLA-4, VISTA, B7-H2, B7-H3, B7-H4, B7-H6, ICOS, HVEM, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, HHLA2, butyrophilins, and A2aR.

In one aspect the disclosure relates to a method of treating or preventing cancer in a subject, comprising administering to the subject a first agent that specifically binds to RGMb conjointly with a second agent that specifically binds to PD-1 or PD-L1. In one aspect of the disclosure the first agent is an antibody, preferably wherein the first agent is an antibody that disrupts RGMb, preferably wherein the antibody that disrupts RGMb is a monoclonal antibody, preferably wherein the antibody that disrupts RGMb is a humanized antibody, preferably wherein the antibody that disrupts RGMb is a bispecific antibody. In one aspect of the disclosure the second agent is an antibody, preferably the second agent is an antibody that blocks PD-1, preferably the antibody that blocks PD-1 is a monoclonal antibody, a humanized antibody, a bispecific antibody, preferably wherein the antibody that blocks PD-1 is selected from cemiplimab (REGN2810), nivolumab (BMS-936558, MDX-1106, ONO-4538), pembrolizumab (MK-3475, SCH 900475), SHR1210, sintilimab (IBI308), spartalizumab (PDR001), tislelizumab (BGB-A317), pidilizumab, BCD-100, toripalimab (JS001), PF-06801591, AB122, AK105, AMG 404, BCD-100, BI 754091, F520, HLX10, HX008, JTX-4014, LZM009, MEDI0680, MGA012, Sym021, TSR-042, PSB205, MGD019, MGD013, AK104, XmAb20717, RO7121661, and CX-188. In one aspect of the disclosure the second agent is an antibody that blocks PD-L1, preferably wherein the antibody that blocks PD-L1 is a monoclonal antibody, preferably the antibody that blocks PD-L1 is a humanized antibody, preferably wherein the antibody that blocks PD-L1 is a bispecific antibody.

In one aspect of the disclosure the antibody that blocks PD-L1 is selected from atezolizumab (MPDL3280A, RG7446, RO5541267), durvalumab (MEDI4736, MEDI-4736), avelumab (MSB0010718C), FS118, BCD-135, BGB-A333, CBT-502, CK-301, CS1001, FAZ053, HLX20, KN035, MDX-1105, MSB2311, SHR-1316, TG-1501, ZKAB001, INBRX-105, MCLA-145, KN046, M7824, and LY3415244. In one aspect of the disclosure the subject is nonresponsive to immune checkpoint inhibitor therapy, or/and wherein the subject is nonresponsive to anti-PD-1 therapy or anti-PD-L1 therapy. In one aspect of the disclosure at least one antibiotic was administered to the subject within a period of time prior to administration of the first agent. In one aspect of the disclosure the at least one antibiotic is Ampicillin, Vancomycin, Neomycin, Metronidazole. In one aspect of the disclosure at least two antibiotics are administered to the patient within the period of time prior to administration of the first agent. In one aspect of the disclosure at least three antibiotics are administered to the patient within the period of time prior to administration of the first agent. In one aspect of the disclosure at least four antibiotics are administered to the patient within the period of time prior to administration of the first agent. In one aspect of the disclosure the period of time is 1 week, 3 weeks, 1 month, 3 months, or 6 months.

In one aspect of the disclosure the subject has dysbiosis. In one aspect of the disclosure the gastrointestinal microbiota of the subject comprises high levels of E. coli or bacteria from phyla that is not Bacteroidetes and Firmicutes. In one aspect of the disclosure the second agent is administered orally, parenterally, systemically or intravenously. In one aspect of the disclosure the cancer is lung cancer, a breast cancer, a colon cancer, a cervical cancer, a pancreatic cancer, a renal cancer, a stomach cancer, a GI cancer, a liver cancer, a bone cancer, a hematological cancer, a neural tissue cancer, a melanoma, a thyroid cancer, a ovarian cancer, a testicular cancer, a prostate cancer, a cervical cancer, a vaginal cancer, or a bladder cancer. In one aspect of the disclosure the cancer comprises a tumor. In one aspect of the disclosure the tumor is an adenocarcinoma, an adrenal tumor, an anal tumor, a bile duct tumor, a bladder tumor, a bone tumor, a brain/CNS tumor, a breast tumor, a cervical tumor, a colorectal tumor, an endometrial tumor, an esophageal tumor, an Ewing tumor, an eye tumor, a gallbladder tumor, a gastrointestinal, a kidney tumor, a laryngeal or hypopharyngeal tumor, a liver tumor, a lung tumor, a mesothelioma tumor, a multiple myeloma tumor, a muscle tumor, a nasopharyngeal tumor, a neuroblastoma, an oral tumor, an osteosarcoma, an ovarian tumor, a pancreatic tumor, a penile tumor, a pituitary tumor, a primary tumor, a prostate tumor, a retinoblastoma, a Rhabdomyosarcoma, a salivary gland tumor, a soft tissue sarcoma, a melanoma, a metastatic tumor, a basal cell carcinoma, a Merkel cell tumor, a testicular tumor, a thymus tumor, a thyroid tumor, a uterine tumor, a vaginal tumor, a vulvar tumor, or a Wilms tumor. In one aspect of the disclosure an additional agent is used, preferably wherein the additional agent is a chemotherapeutic agent, or wherein the additional agent is an additional immune checkpoint inhibitor, preferably wherein the additional immune checkpoint inhibitor comprises an antibody specific for an immune checkpoint protein selected from CTLA-4, VISTA, B7-H2, B7-H3, B7-H4, B7-H6, ICOS, HVEM, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, HHLA2, butyrophilins, and A2aR. In one aspect the disclosure concerns a method of treating or preventing cancer in a subject, comprising administering to the subject a first agent that disrupts the interaction between PD-L2/RGMb and a second agent that disrupts the interaction between PD-1/PD-L1, preferably wherein the first agent is an antibody, or wherein the first agent is an antibody that blocks or disrupts PD-L2, preferably wherein the antibody that blocks or disrupts PD-L2 is a monoclonal antibody, or/and a humanized antibody, or/and a bispecific antibody. In one aspect the first agent is an antibody that disrupts RGMb, preferably a monoclonal antibody, or/and a humanized antibody, or/and a bispecific antibody. In one aspect the second agent is an antibody, preferably wherein the second agent is an antibody that blocks PD-1, preferably the antibody that blocks PD-1 is a monoclonal antibody, or/and the antibody that blocks PD-1 is a humanized antibody, or/and wherein the antibody that blocks PD-1 is a bispecific antibody. In one aspect the antibody that blocks PD-1 is selected from cemiplimab (REGN2810), nivolumab (BMS-936558, MDX-1106, ONO-4538), pembrolizumab (MK-3475, SCH 900475), SHR1210, sintilimab (IBI308), spartalizumab (PDR001), tislelizumab (BGB-A317), pidilizumab, BCD-100, toripalimab (JS001), PF-06801591, AB122, AK105, AMG 404, BCD-100, BI 754091, F520, HLX10, HX008, JTX-4014, LZM009, MEDI0680, MGA012, Sym021, TSR-042, PSB205, MGD019, MGD013, AK104, XmAb20717, RO7121661, and CX-188. In one preferred aspect of the disclosure the second agent is an antibody that blocks PD-L1, preferably wherein the antibody that blocks PD-L1 is a monoclonal antibody, or/and wherein the antibody that blocks PD-L1 is a humanized antibody, or/and wherein the antibody that blocks PD-L1 is a bispecific antibody. In one aspect of the disclosure the antibody that blocks PD-L1 is selected from atezolizumab (MPDL3280A, RG7446, RO5541267), durvalumab (MEDI4736, MEDI-4736), avelumab (MSB0010718C), FS118, BCD-135, BGB-A333, CBT-502, CK-301, CS1001, FAZ053, HLX20, KN035, MDX-1105, MSB2311, SHR-1316, TG-1501, ZKAB001, INBRX-105, MCLA-145, KN046, M7824, and LY3415244. In one aspect of the disclosure the subject is nonresponsive to immune checkpoint inhibitor therapy, or wherein the subject is nonresponsive to anti-PD-1 therapy or anti-PD-L1 therapy. In one aspect of the disclosure at least one antibiotic was administered to the subject within a period of time prior to administration of the first agent, preferably wherein the at least one antibiotic is Vancomycin, or Neomycin, or Metronidazole, or Ampicillin, preferably wherein at least two antibiotics are administered to the patient within the period of time prior to administration of the first agent, or wherein at least three antibiotics are administered to the patient within the period of time prior to administration of the first agent, or wherein at least four antibiotics are administered to the patient within the period of time prior to administration of the first agent, preferably wherein the period of time is 1 or 3 weeks, or 1, 3 or 6 months. In one aspect of the disclosure the subject has dysbiosis, or/and wherein the gastrointestinal microbiota of the subject comprises high levels of E. coli or bacteria from other phyla that is not Bacteroidetes and Firmicutes. In one aspect of the disclosure the first agent is administered or used systemically, orally, parenterally, or intravenously.

In a preferred aspect of the disclosure the second agent is administered or used intravenously or parenterally or orally or systemically. In one aspect of the disclosure the cancer is lung cancer, a breast cancer, a colon cancer, a cervical cancer, a pancreatic cancer, a renal cancer, a stomach cancer, a GI cancer, a liver cancer, a bone cancer, a hematological cancer, a neural tissue cancer, a melanoma, a thyroid cancer, a ovarian cancer, a testicular cancer, a prostate cancer, a cervical cancer, a vaginal cancer, or a bladder cancer, or/and wherein the cancer comprises a tumor. In a preferred aspect the tumor is an adenocarcinoma, an adrenal tumor, an anal tumor, a bile duct tumor, a bladder tumor, a bone tumor, a brain/CNS tumor, a breast tumor, a cervical tumor, a colorectal tumor, an endometrial tumor, an esophageal tumor, an Ewing tumor, an eye tumor, a gallbladder tumor, a gastrointestinal, a kidney tumor, a laryngeal or hypopharyngeal tumor, a liver tumor, a lung tumor, a mesothelioma tumor, a multiple myeloma tumor, a muscle tumor, a nasopharyngeal tumor, a neuroblastoma, an oral tumor, an osteosarcoma, an ovarian tumor, a pancreatic tumor, a penile tumor, a pituitary tumor, a primary tumor, a prostate tumor, a retinoblastoma, a Rhabdomyosarcoma, a salivary gland tumor, a soft tissue sarcoma, a melanoma, a metastatic tumor, a basal cell carcinoma, a Merkel cell tumor, a testicular tumor, a thymus tumor, a thyroid tumor, a uterine tumor, a vaginal tumor, a vulvar tumor, or a Wilms tumor. In an aspect an additional agent is administered and is a chemotherapeutic agent, or wherein the additional agent is an additional immune checkpoint inhibitor, preferably wherein the additional immune checkpoint inhibitor comprises an antibody or agent specific for an immune checkpoint protein selected from CTLA-4, VISTA, B7-H2, B7-H3, B7-H4, B7-H6, ICOS, HVEM, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, HHLA2, butyrophilins, and A2aR.

## Claims

1. A method of converting a non-responder to an anti-PD-Ll or anti-PD-1 cancer therapy to a responder, comprising administering to the non-responder, in combination with the anti-PD-1 or anti-PD-Ll cancer therapy, a synergistically effective amount of an anti-PD-L2 antibody.

2. The method of claim 1, wherein the non-responder has exhibited resistance to the anti-PD-L1 or anti-PD-1 cancer therapy caused or suspected to be caused by dysbiosis.

3. The method of claim 2, wherein the dysbiosis is caused by any one or more of the conditions selected from intestinal infections, ulcerative colitis, Crohn's disease, irritable bowel syndrome, colon cancer, significant changes in diet, or a combination thereof.

4. The method of any one of claims 1-3, wherein the non-responder has any one or more symptoms selected from diarrhea, constipation, abdominal cramping, loose stool, abnormal amounts of gas or bloating, or a combination thereof.

5. The method of any one of claims 1-4, wherein at least one antibiotic is administered to the non-responder within a period of time prior to administration of the anti-PD-Ll or anti-PD-1 cancer therapy.

6. The method of claim 5, wherein the at least one antibiotic is Vancomycin, Neomycin, Metronidazole, Ampicillin, or a combination thereof.

7. The method of claim 5 or 6, wherein the period of time is at least 1 week.

8. The method of any one of claims 1-7, wherein a gastrointestinal microbiota of the non-responder comprises high levels of E. coli or bacteria from phyla that is not Bacteroidetes and Firmicutes.

9. The method of any one of claims 1-8, wherein the anti-PD-L2 antibody is administered for at least one week.

10. The method of any one of claims 1-9, wherein the anti-PD-L2 antibody is administered intraperitoneally, subcutaneously, intramuscularly, intravenously, intrathecally, intracerebrally, orally, nasally, rectally, intravaginally, parenterally, intracisternally, topically, buccally or sublingually.

11. The method of any one of claims 1-10, wherein the cancer therapy is for the treatment of a cancer comprising a solid tumor.

12. The method of any one of claims 1-11, wherein the cancer therapy is for the treatment of an adenocarcinoma or a colorectal tumor.

13. The method of any one of claims 1-12, wherein administration of the anti-PD-L2 antibody in combination with the anti-PD-1 or anti-PD-Ll therapy results in a synergistic reduction in tumor volume.

14. The method of any one of claims 1-13, wherein the non-responder is a non-responder to the anti-PD-1 cancer therapy.

15. The method of any one of claims 1-14, wherein the non-responder is a non-responder to the anti-PD-L1 cancer therapy.
